(19) Europäisches Patentamt
European Patent Office
Office européen des brevets

(11) EP 4 159 190 A1

(12) **EUROPEAN PATENT APPLICATION**
published in accordance with Art. 153(4) EPC

(43) Date of publication:
**05.04.2023 Bulletin 2023/14**

(21) Application number: **21818869.6**

(22) Date of filing: **27.05.2021**

(51) International Patent Classification (IPC):
***A61K 8/89*** *(2006.01)* ***A61Q 5/00*** *(2006.01)*
***A61Q 5/06*** *(2006.01)*

(52) Cooperative Patent Classification (CPC):
**A61K 8/58; A61K 8/89; A61K 8/891; A61K 8/898; A61Q 5/00; A61Q 5/06; A61Q 5/10**

(86) International application number:
**PCT/JP2021/020130**

(87) International publication number:
**WO 2021/246272 (09.12.2021 Gazette 2021/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.06.2020 JP 2020095661**

(71) Applicant: **Kao Corporation**
**Chuo-ku**
**Tokyo 103-8210 (JP)**

(72) Inventor: **MAEKAWA, Tomoka**
**Tokyo 131-8501 (JP)**

(74) Representative: **Hoffmann Eitle**
**Patent- und Rechtsanwälte PartmbB**
**Arabellastraße 30**
**81925 München (DE)**

(54) **COSMETIC COMPOSITION**

(57) A cosmetic composition containing the following components (A) to (C): (A) a silicone film-forming agent, (B) a high-molecular-weight organopolysiloxane, (C) an organopolysiloxane having a polyalkylene oxide moiety and a cationic group, other than the component (A) and the component (B).

EP 4 159 190 A1

## Description

Field of the Invention

The present invention relates to a cosmetic composition.

Background of the Invention

It is known that hair is damaged and difficult to handle by external factors in daily life, and chemical treatment such as perm and color. In order to solve such a situation, a conditioning agent or a styling agent is used to impart gloss or smoothness to hair. However, many of these effects are lost by one shampooing and by physical forces applied in daily life such as friction between hair and a pillow or the like during sleeping, and products capable of sustaining the effects are desired. Further, It is desired that a good feel can be imparted to hair in any daily life, i.e., in a dry state or a wet state. For example, Patent Literature 1 (JP 2015-515981 T) discloses that a composition containing a continuous aqueous phase, and a discontinuous phase that contains a plasticized MQ-type silicone resin and/or a derivative thereof, and/or a plasticized MT-type silicone resin and/or a derivative thereof, in which the discontinuous phase is emulsified in the aqueous phase, is useful as a haircare product for treating split ends.

Patent Literature 2 (JP 2016-503433 T) discloses a composition containing a silicone resin and a silicone rubber, in which a mixture of the silicone resin and the silicone rubber have a softening point higher than 50°C and an elastic modulus of 106 Pa or less at an environmental temperature, and discloses that when a formulation containing the composition is applied to hair, it can impart a smooth feel and a strong gloss to the hair.

Regarding a cosmetic composition containing a colorant such as a pigment, a technique of using a film forming agent for the purpose of increasing durability after application and suppressing colorant transfer is investigated. For example, Patent Literature 3 (JP 2000-501074 T) discloses a cosmetic composition containing a mixture prepared by mixing an organosiloxane resin and a fluid diorganopolysiloxane polymer in a specific ratio, and a volatile carrier, and discloses formation of a thin durable film having resistance to transfer in contact with a substance such as cloth or a towel.

As one hair dyeing technique, there is known a temporary hair dye technique of using a pigment as a colorant and forming a film that contains the colorant on hair for dyeing the hair. The hair dye can dye hair in a simple manner with few damages to hair, but some may easily discolor by shampooing, or owing to poor rubfastness after hair dyeing, the color film may peel off by friction given thereto to cause secondary adhesion to clothes and pillows.

Regarding this, for example, Patent Literature 4 (JP H10-265354 A) discloses that a hair dye containing a volatile oil, a water-repellent polymer such as silicone resin and a high-molecular-weight organopolysiloxane dissolving in the volatile oil, a powder, and a nonvolatile oil compatible with the volatile oil, in which at least a part of the powder is a color pigment, is applicable in a simple manner and secures good color duration not causing secondary adhesion after application.

Summary of the Invention

The present invention relates to the following [1] to [9].

[1] A cosmetic composition containing the following components (A) to (C),

(A) a silicone film-forming agent,
(B) a high-molecular-weight organopolysiloxane,
(C) an organopolysiloxane having a polyalkylene oxide moiety and a cationic group, other than the component (A) and the component (B).

[2] The cosmetic composition according to the above [1], which is a hair cosmetic composition.
[3] The cosmetic composition according to the above [1], which is a hair dye composition.
[4] A method for treating a keratin substance, including a step of applying the cosmetic composition of the above [1] to a keratin substance and then drying it.
[5] A method for treating hair, including a step of applying the hair cosmetic composition of the above [2] to hair and then drying it.
[6] A method for dyeing hair, including a step of applying the hair dye composition of the above [3] to hair and then drying it.
[7] A cosmetic kit provided with at least two compositions, wherein:
the cosmetic composition obtained by mixing all the compositions constituting the cosmetic kit contains the components (A) to (C).

[8] A method for forming a film on the surface of a keratin substance, including the following step (I) to step (III) in that order,

Step (I): a step of temporarily compatibilizing or dispersing a composition containing the following components (A) to (C),

(A) a silicone film-forming agent,
(B) a high-molecular-weight organopolysiloxane,
(C) an organopolysiloxane having a polyalkylene oxide moiety and a cationic group, other than the component (A) and the component (B),

Step (II): a step of applying the composition prepared in the step (I) to the surface of a keratin substance,
Step (III): a step of drying the composition applied to the surface of a keratin substance in the step (II).

[9] A film to cover a surface of a keratin substance, wherein:
the film is a multilayer-structured film containing the components (A) to (C), having a layer containing the component (B) in the outermost surface of the film, and having a layer containing the component (C) on the interface side to the keratin substance.

## Brief Description of the Drawing

Fig. 1 shows an elastic modulus analyzing image of a hair cross section after treatment with the hair cosmetic composition of Example 1, and an enlarged image of the film part thereof.

## Detailed Description of the Invention

"Polymer" used in the present specification means a compound corresponding to a repetition of one or plural units (these units are derived from a compound known as a monomer). This or these units are repeated at least two times preferably at least three times.
"Hair" used in the present specification means mainly head hair.
"Hydrophobic" used in the present specification means that a solubility in water of a substance is less than 1% by mass at 25°C.
"Film formation" used in the present specification means that, when applied to a substrate, a film is left thereon.
"Volatile" used in the present specification means a substance having a boiling point of 260°C or lower under normal pressure.

[Cosmetic Composition]

The cosmetic composition of the present invention contains the following components (A) to (C).

(A) A silicone film-forming agent.
(B) A high-molecular-weight organopolysiloxane.
(C) An organopolysiloxane having a polyalkylene oxide moiety and a cationic group, other than the component (A) and the component (B).

Having the above constitution, the cosmetic composition of the present invention is, when applied to a keratin substance such as skin or hair, able to impart a good feel thereto in any of a dry state or a wet state, excellent in durability to friction, and excellent in sustainability of these effect even after washing.
Patent Literature 1 investigates sustainability to cycles of washing, blow drying and hot ironing, but does not refer to improvement of feel of hair in a wet state (for example, in shampooing), and to durability to friction against pillows.
Patent Literature 2 does not refer to improvement of feel of hair in a wet state, durability against friction, and durability against washing and friction of these effects.
Patent Literature 3 investigates resistance to transfer, but does not refer to durability against washing of the effect and feel in a dry state and a wet state.
Patent Literature 4 refers to secondary adhesion of a hair dye, color sustainability and feel in use, but does not refer to improvement of feel of hair in a wet state.
Among such hitherto-existing cosmetic compositions, there could not as yet be provided cosmetic compositions which, when applied to a keratin substance such as skin or hair, can impart a good feel in any of a dry state and a wet

state, and are excellent in durability to friction, and excellent in sustainability of these effects even after washing.

An object of the present invention is to provide a cosmetic composition which, when applied to a keratin substance such as skin or hair, can impart thereto a good feel in any of a dry state and a wet state, and is excellent in durability to friction, and excellent in sustainability of these effects even after washing.

It is considered that the film formed on a keratin substance for solving the problems is required to have the following properties.

It is considered that, for imparting a good feel to a keratin substance, the film surface needs to be smooth, and for durability to friction, the film needs to be adhesive to a keratin substance and needs to have a film strength durable to the physical force during friction. Further, for securing durability to washing, it is considered that the film needs to be hydrophobic and to have a film strength durable to the physical force during washing.

Namely, for solving the problems with the present invention, it is considered that plural different properties that the film formed on a keratin substance is hydrophobic and that the film has a smooth surface, a high film strength and a high adhesiveness to a keratin substance must be satisfied all at the same time. Accordingly, another object of the present invention is to form a film on a keratin substance, which satisfies all the requirements that the film is hydrophobic and has a smooth surface, a high film strength and a high adhesiveness to a keratin substance.

The present inventor has found that a cosmetic composition containing a silicone film-forming agent, a high-molecular-weight organopolysiloxane, and an organopolysiloxane having a polyalkylene oxide moiety and a cationic group can solve the above-mentioned problems, and has completed the present invention.

According to the present invention, there can be provided a cosmetic composition which, when applied to a keratin substance such as skin or hair, can impart thereto a good feel in any of a dry state and a wet state, and is excellent in durability to friction, and excellent in sustainability of these effects even after washing. In the case where the cosmetic composition is used as a hair dye composition, it can impart a good feel to hair in any of a dry state and a wet state, and the color film peels little by friction after hair dyeing, and can suppress secondary adhesion to clothes and pillows. In addition, after shampooing, discoloration is low and color sustainability is good, and further, even after shampooing, a good feel retains and a secondary adhesion suppressing effect can also be sustained.

The reason why the cosmetic composition of the present invention exhibits the above-mentioned effects is, though not clear, presumed as follows.

The cosmetic composition containing the component (A), the component (B) and the component (C) of the present invention is, when applied to the surface of a keratin substance, able to form a hydrophobic film.

Preferably, the cosmetic composition containing the component (A), the component (B) and the component (C) is temporarily compatibilized or dispersed before use, and then applied to the surface of a keratin substance, whereby it is considered that the components could be in phase separation with time and a multilayered hydrophobic film having a concentration distribution of the components in the thickness direction could be formed on the surface of a keratin substance.

A specific multilayered structure is a multilayered structure having at least two layers having a layer containing the component (B) in the outermost surface of the film, and having a layer containing the component (C) on the interface side to the keratin substance, and is preferably a multilayered structure having at least three layers that further has a layer containing the component (A) between the layer containing the component (B) and the layer containing the component (C).

Here, the constituent layers may not be distinctly separated from each other. In the present specification, the "layer" in the multilayered structure may have a region which has a concentration distribution in the thickness direction of the film and in which at least one component of the components (A) to (C) is eccentrically located as a main component, and such region is referred to as "layer" herein. For example, "having a layer containing the component (B) in the outermost surface of the film" means that in the film, the component (B) is eccentrically located as a main component on the outermost surface side relative to the thickness direction of the film. The main component as referred to herein means a component having a highest concentration among the components (A) to (C). With that, it is considered that a high-molecular chain of a component mainly contained in the neighboring layer may mutually invade into the interlayer to form an interfacial phase having a thickness and having a continuously varying concentration.

In the case where the cosmetic composition of the present invention is applied to the surface of a keratin substance, the component (B) is considered to be eccentrically located in the outermost surface of the formed film and play a role in imparting a good feel to the surface of a keratin substance, and further play a function as a surface protective layer. The component (C) is considered to be eccentrically located on the side of the keratin substance, and is considered to firmly adsorb the formed film to the surface of the keratin substance to exhibit the film peeling preventing effect.

On the other hand, the component (A) is a hydrophobic film-forming agent, and is considered to exist in the layer containing the component (B) or the component (C) in the formed film, or to be eccentrically located in the intermediate part of the layer containing the component (B) and the component (C) in the formed film to thereby secure the strength and the durability of the entire formed film and to also exhibit the durability improving effect of various characteristics given to the keratin substance owing to the synergistic effect with the component (B) and the component (C).

In the case where a functional powder to be mentioned hereinunder is incorporated in the cosmetic composition, the functional powder can be kept in the film to enhance various functions and sustainability thereof.

Owing to the mechanism of the action mentioned above, it is considered that, in the case where the cosmetic composition is, for example, a hair cosmetic composition, because of synergistic effect of the film-forming performance of the component (A), the feel-imparting effect and the surface protective performance of the component (B) and the film peeling preventing performance of the component (C), when the composition is applied to hair, it can impart a good feel to hair in any of a dry state and a wet state, and can provide durability to friction and sustainability of these effects after washing.

In addition, it is also considered that, in the case where the cosmetic composition is a hair dye composition such as a temporary hair dye composition or a semi-permanent hair dye composition, it can be a hair dye composition capable of imparting a good feed to hair in any of a dry state and a wet state after hair dyeing, and capable of suppressing the colored film from peeling owing to friction after hair dyeing. Moreover, it is considered that the hair dye composition can secure good color retentiveness with little discoloration after shampooing, can secure a good feel even after shampooing and can secure a secondary adhesion preventing effect.

The mechanism of the action of the present invention is not limited to the above.

Components contained in the cosmetic composition of the present invention are described below.

<Component (A): silicone film-forming agent>

The cosmetic composition of the present invention contains a silicone film-forming agent as the component (A). Containing the component (A), the cosmetic composition of the present invention is, when applied to a keratin substance such as skin or hair, able to form a hydrophobic film having high durability to friction and high sustainability of various effects after washing. In the case where a functional powder to be mentioned hereinunder is blended in the cosmetic composition, the functional powder can be held in the film to improve various functions and sustainability thereof.

As the component (A), a silicone film-forming agent usable in ordinary cosmetic materials can be used, and is preferably solid at 25°C.

From the viewpoint of improving film formability and durability, the component (A) is preferably at least one selected from the group consisting of the following components (A1) and (A2).

(A1) A silicone resin represented by an average formula, $(R^1)_mSiO_{(4-m)/2}$

wherein $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3,
which contains at least one unit selected from the group consisting of a T unit represented by $R^1SiO_{3/2}$ and a Q unit represented by $SiO_{4/2}$.

(A2) A silicone polymer containing a polysiloxane moiety and a moiety formed of a non-silicone organic chain.

Preferably, the component (A1) includes those containing at least one selected from the group consisting of the following component (A1-1) and component (A1-2).

(A1-1) A silicone resin represented by the above-mentioned average formula, containing a T unit represented by $R^1SiO_{3/2}$ and substantially not containing a Q unit represented by $SiO_{4/2}$.
(A1-2) A silicone resin represented by the above-mentioned average formula, and containing a Q unit represented by $SiO_{4/2}$ and an M unit represented by $(R^1)_3SiO_{1/2}$.

Preferably, the component (A2) includes those containing at least one selected from the group consisting of the following component (A2-1) to (A2-4).

(A2-1) An acryl silicone polymer.
(A2-2) A silicone-modified alicyclic structure-containing polymer.
(A2-3) A silicone-modified pullulan.
(A2-4) A polyurea/urethane silicone.

(Component (A1))

The component (A1) is a silicone resin represented by an average formula, $(R^1)_mSiO_{(4-m)/2}$

wherein $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3,

which contains at least one unit selected from the group consisting of a T unit represented by $R^1SiO_{3/2}$ and a Q unit represented by $SiO_{4/2}$.

The component (A1) is represented by the above-mentioned average formula and contains at least one unit selected from the above-mentioned T unit and Q unit, and therefore has a crosslinked structure in the molecule. Having the structure, the silicone resin is considered to be able to form a film having higher durability. The component (A1) does not contain a polyorganosiloxane cured product powder which is infusible and does not have a softening point and which is generally insoluble in an organic solvent.

In the average formula, $R^1$ represents a hydrocarbon group having 1 or more and 12 or less carbon atoms and optionally substituted with fluorine, or a hydroxy group. The carbon number of the hydrocarbon group is, from the viewpoint of improving film formability and durability, 1 or more and preferably 9 or less, more preferably 6 or less, even more preferably 4 or less.

The hydrocarbon group may be any of an aliphatic group or an aromatic group, and examples thereof include an alkyl group, an alkenyl group, an aryl group and an aralkyl group. The alkyl group and the alkenyl group may be linear or branched.

Among the above, from the viewpoint of availability and stability, the hydrocarbon group is preferably an alkyl group, an aryl group or an aralkyl group.

The alkyl group includes a methyl group, an ethyl group, an n-propyl group, an isopropyl group, various butyl groups, various pentyl groups, various hexyl groups, various heptyl groups, various octyl groups, various nonyl group, various decyl groups, various undecyl groups, and various dodecyl groups. The word "various" means a linear or branched hydrocarbon group, and for example, "various butyl groups" include "an n-butyl group, a sec-butyl group, an isobutyl group and a tert-butyl group".

The aryl group includes a phenyl group, a toluyl group, a dimethylphenyl group, and a naphthyl group, and is preferably a phenyl group.

The aralkyl group includes a benzyl group, a phenylethyl group, a phenylpropyl group, and a phenylbutyl group, and is preferably a phenylpropyl group.

In the case where $R^1$ is substituted with fluorine, at least one hydrogen atom of the hydrocarbon group may be substituted with a fluorine atom.

$R^1$ is, from the viewpoint of improving film formability and durability, preferably an optionally fluorine-substituted, alkyl group having 1 or more and 12 or less carbon atoms, aryl group having 6 or more and 12 or less carbon atoms or aralkyl group having 7 or more and 12 or less carbon atoms, more preferably an optionally fluorine-substituted, alkyl group having 1 or more and 8 or less carbon atoms or phenyl group, even more preferably an optionally fluorine-substituted, alkyl group having 1 or more and 6 or less carbon atoms or phenyl group. The fluorine-substituted alkyl group is preferably a group represented by $CF_3$-R-wherein R represents an alkylene group having 2 or more and 7 or less carbon atoms, preferably 2 or more and 5 or less carbon atoms.

$R^1$ is more preferably a trifluoropropyl group, an alkyl group having 1 or more and 4 or less carbon atoms, or a phenyl group, even more preferably a trifluoropropyl group, a methyl group, an ethyl group, an n-propyl group, an isopropyl group or an n-butyl group, further more preferably a trifluoropropyl group, a methyl group or an n-propyl group, and further more preferably a methyl group.

The component (A1) may contain at least one unit selected from the group consisting of a T unit represented by $R^1SiO_{3/2}$ and a Q unit represented by $SiO_{4/2}$ and, from the viewpoint of improving film formability and durability, preferably further contains at least one unit selected from the group consisting of an M unit represented by $(R^1)_3SiO_{1/2}$ and a D unit represented by $(R^1)_2SiO_{2/2}$. $R^1$ is the same as above.

From the viewpoint of improving film formability and durability, the component (A1) is preferably at least one selected from the group consisting of a silicone resin (A1-1) represented by the above-mentioned average formula, containing a T unit represented by $R^1SiO_{3/2}$ and substantially not containing a Q unit represented by $SiO_{4/2}$, and a silicone resin (A1-2) represented by the above-mentioned average formula, and containing a Q unit represented by $SiO_{4/2}$ and an M unit represented by $(R^1)_3SiO_{1/2}$.

[Silicone Resin (A1-1)]

The silicone resin (A1-1) represented by the above-mentioned average formula, containing a T unit represented by $R^1SiO_{3/2}$ and substantially not containing a Q unit represented by $SiO_{4/2}$ (hereinafter also referred to as "component (A1-1)") is a silicone resin containing a T unit and may contain an M unit and a D unit, and is preferably a silicone resin represented by $[R^1SiO_{3/2}]_a[(R^1)_3SiO_{1/2}]_b$ wherein a and b each are an average repeating unit number, and a>0 and b≥0.

The wording "substantially not containing XX" means the constituent ratio of XX in the silicone resin is less than 1 mol%.

$R^1$ is the same as above, and is preferably an alkyl group having 1 or more and 4 or less carbon atoms or a phenyl group, more preferably a methyl group, an ethyl group, an n-propyl group or an isopropyl group, even more preferably a methyl group, an n-propyl group or an isopropyl group.

The component (A1-1) includes polysilsesquioxanes such as polymethylsilsesquioxane, polypropylsilsesquioxane, polyphenylsilsesquioxane, polymethylphenylsilsesquioxane, and fluorine-modified alkyldimethylpolysilsesquioxanes, and among these, one or more can be used. Fluorine-modified alkyldimethylpolysilsesquioxanes include, as INCI nomenclature, trifluoropropyldimethylsiloxy/trimethylsiloxy)silsesquioxane.

Above all, from the viewpoint of improving film formability and durability, the component (A1-1) is preferably at least one selected from the group consisting of polymethylsilsesquioxane and polypropylsilsesquioxane.

Commercial products of the component (A1-1) include SilForm Flexible Resin (polymethylsilsesquioxane), SilForm FR-5 (polydimethylsiloxane solution of (trifluoropropyldimethylsiloxy/trimethylsiloxy)silsesquioxane) (all by Momentive Performance Materials Corporation), DOWSIL 680 ID Fluid (isododecane solution of 75 mass% polypropylsilsesquioxane) (by Day Toray Corporation), SR-21 (polyphenylsilsesquioxane), SR-23 (polyphenylsilsesquioxane), SR-33 (polymethylphenylsilsesquioxane) (all by Konishi Chemical Industry Co., Ltd.).

[Silicone Resin (A1-2)]

The silicone resin (A1-2) represented by the above-mentioned average formula, and containing a Q unit represented by $SiO_{4/2}$ and an M unit represented by $(R^1)_3SiO_{1/2}$ (hereinafter also referred to as "component (A1-2)" is a silicone resin substantially containing a Q unit and an M unit and optionally containing a D unit or a T unit, and is preferably a silicone resin represented by $[SiO_{4/2}]_c[(R^1)_3SiO_{1/2}]_d$ wherein c and d each are an average repeating unit number and c>0 and d>0.

$R^1$ is the same as above, and is preferably an optionally-fluorine substituted, alkyl group having 1 or more and 6 or less carbon atoms or phenyl group, more preferably a trifluoropropyl group, an alkyl group having 1 or more and 4 or less carbon atoms, or a phenyl group, even more preferably a trifluropropyl group, methyl group, an ethyl group, an n-propyl group, or an isopropyl group, further more preferably a trifluoropropyl group or a methyl group, and further more preferably a methyl group.

The component (A1-2) includes trimethylsiloxysilicate, phenylpropyldimethylsiloxysilicate, fluorine-modified alkyldimethylsiloxysilicates, and crosspolymers produced by crosslinking these siloxysilicates with dimethiconol, and at least one of these can be used. Fluorine-modified alkyldimethylsiloxysilicates include trifluoroalkyldimethyltrimethylsiloxysilicate, such as trifluoropropyldimethyltrimethylsiloxysilicate of, as ICNI nomenclature, trifluoropropyldimethyl/trimethylsiloxysilicate. Crosspolymers produced by crosslinking siloxysilicates with dimethiconol include, as ICNI nomenclature, (trimethylsiloxysilicate/dimethiconol) crosspolymer.

Above all, from the viewpoint of improving film formability and durability, the component (A1-2) is preferably at least one selected from the group consisting of trimethylsiloxysilicate, trifluoropropyldimethyltrimethylsiloxysilicate and (trimethylsiloxysilicate/dimethiconol) crosspolymer, more preferably at least one selected from the group consisting of trimethylsiloxysilicate, and trifluoropropyldimethyltrimethylsiloxysilicate, even more preferably trimethylsiloxy silicate.

Commercial products of trimethylsiloxysilicate of the component (A1-2) include KF-7312J (50 mass% decamethylcyclopentasiloxane solution), KF-9021 (50 mass % decamethylcyclopentasiloxane solution), X-21-5249(50 mass % decamethylcyclopentasiloxane solution), X-21-5595 (60 mass % isododecane solution), and X-21-5616 (60 mass % isododecane solution) (all by Shin-Etsu Chemical Industry Co., Ltd.), SS4267 (35 mass % dimethylpolysiloxane solution), SR1000, SS4230 (45 mass % cyclopentasiloxane solution), SS4267 (35 mass % dimethylpolysiloxane solution), and Silsoft 74 (75 mass %isododecane solution) (all by Momentive Performance Materials Corporation), BY11-018 (30 mass% cyclopentasiloxane solution), and MQ-1600 Solid Resin (all by Dow Toray Corporation), and BELSIL TMS 803 (by Wacker Asahi Kasei Silicone Co., Ltd.).

Commercial products of phenylpropyldimethylsiloxysilicate include SilShine 151 (by Momentive Performance Materials Corporation).

Commercial products of fluorine-modified alkyldimethylsiloxysilicates include, XS66-B8226 (50 mass% cyclopentasiloxane solution), XS66-C1191, and XS66-B8636 (50 mass % dimethicone solution) (all by Momentive Performance Materials Corporation), as INCI nomenclature, (trifluoropropyldimethyl/trimethylsiloxysilicate).

Commercial products of trimethylsiloxysilicate crosspolymer include DOWSIL FC-5002 IDD Resin Gum (40 mass% isododecane solution of (trimethylsiloxysilicate/dimethiconol) crosspolymer) (by Dow Toray Corporation).

(Component (A2))

The component (A2) is a silicone polymer containing a polysiloxane moiety and a moiety formed of a non-silicone organic chain. The non-organic silicone organic monomer to constitute the moiety of a non-silicone organic

chain is, from the viewpoint of availability on the market, preferably selectable from a radicalpolymerizable ethylenically-unsaturated monomer, a polycondensationpolymerizable monomer (e.g., those to form polyamides, polyesters or polyurethanes), and a ring-cleavable monomer (e.g., oxazoline or caprolactone-type ones).

Preferably, the component (A2) includes those containing at least one selected from the group consisting of the following (A2-1) to (A2-4), more preferably those containing the component (A2-1).

(A2-1) An acryl silicone polymer.
(A2-2) A silicone-modified alicyclic structure-containing polymer.
(A2-3) A silicone-modified pullulan.
(A2-4) A polyurea/urethane silicone.

[Acryl Silicone Polymer (A2-1)]

The acryl silicone polymer of the component (A2-1) includes an acrylic polymer having a carbosiloxane dendrimer structure in the side chain, an acryl-silicone graft copolymer, and a graft-type copolymer or alternate block-type copolymer where a structural unit of a polysiloxane group and a structural unit of a polymer of an unsaturated monomer bond via a sulfide bond.

The acrylic polymer having a carbosiloxane dendrimer structure in the side chain includes a silicone dendrimer-acryl copolymer, and for example, can be produced according to the production method described in JP H11-1530 A and JP 2000-63225 A.

The acrylic polymer having a carbosiloxane dendrimer structure in the side chain is preferably, as INCI nomenclature, acrylates/polytrimethylsiloxymethacrylate copolymer. Commercial products thereof include DOWSIL FA 4001 CM Silicone Acrylate (30 mass% decamethylcyclopentasiloxane solution), DOWSIL FA 4002 ID Silicone Acrylate (40 mass% isododecane solution) (all by Dow Toray Corporation).

The acryl-silicone graft copolymer includes a radical polymer of an organopolysiloxane compound having a radical polymerizable group at one terminal of the molecular chain and a radical polymerizable monomer mainly composed of an acrylate and/or a methacrylate.

Examples of the radical polymer of an organopolysiloxane compound having a radical polymerizable group at one terminal of the molecular chain and a radical polymerizable monomer mainly composed of an acrylate and/or a methacrylate usable here include those described in JP H2-25411 A and JP H2-132141 A, and acryl-silicone graft copolymers described in JP H3-162442 A and JP 2003-104825 A.

The acryl-silicone graft copolymer is preferably, as INCI nomenclature, (acrylates/dimethicone) copolymer. Commercial products thereof include KP-545 (30 mass % decamethylcyclopentasiloxane solution), KP-549 (40 mass% methyltrimethicone solution), and KP-550 (40 mass% isododecane solution) (all by Shin-Etsu Chemical Industry Co., Ltd.).

The graft-type copolymer or alternate block-type copolymer where a structural unit of a polysiloxane group and a structural unit of a polymer of an unsaturated monomer bond via a sulfide bond include graft-type copolymers or alternate block-type copolymers described in JP H6-92825 A.

Above all, from the viewpoint of improving film formability and durability, the component (A2-1) is preferably at least one selected from the group consisting of an acrylic polymer having a carbosiloxane dendrimer structure in the side chain and an acryl-silicone graft copolymer, more preferably at least one selected from (acrylates/polytrimethylsiloxymethacrylate) copolymer and (acrylates/dimethicone) copolymer.

[Silicone-Modified Alicyclic Structure-Containing Polymer (A2-2)]

Examples of the silicone-modified alicyclic structure-containing polymer include silicone-modified cyclic polyolefins, and preferred examples thereof include silicone-modified polynorbornenes represented by the following general formula (A2-2-1).

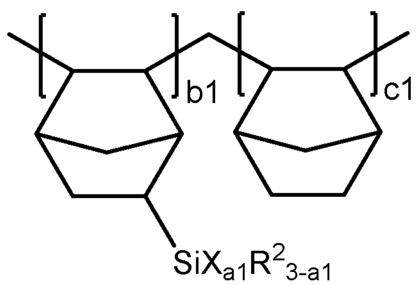

(A2-2-1)

wherein $R^2$ each independently represents an alkyl group having 1 or more and 12 or less carbon atoms, X represents a group represented by the following formula (i), a1 is an integer of 1 or more and 3 or less, b1 and c1 each are a repeating unit number, and are each independently an integer of 1 or more.

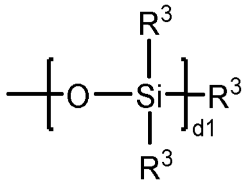
(i)

wherein $R^3$ each independently represents a hydrocarbon group having 1 or more and 12 or less carbon atoms, and d1 is an integer of 1 or more and 5 or less.

In the general formula (A2-2-1), $R^2$ is preferably a methyl group, an ethyl group, an n-propyl group, a butyl group or a pentyl group, more preferably a methyl group.

X is a group represented by the formula (i), and in the formula (i), $R^3$ each are independently a hydrocarbon group having 1 or more and 12 or less carbon atoms. $R^3$ is preferably an alkyl group having 1 or more and 12 or less or a phenyl group, more preferably an alkyl group having 1 or more and 3 or less carbon atoms, even more preferably a methyl group. d1 is an integer of 1 or more and 5 or less, and is, from the viewpoint of versatility, preferably d1 = 1. Specifically, X is preferably a trimethylsiloxy group.

a1 is an integer of 1 or more and 3 or less, and, for example, in the polymer, a repeating unit of a1 = 2 and a repeating unit of a1 = 3 may exist as mixed. From the viewpoint of versatility, a1 is preferably 3.

The proportion of b1 and c1 in the general formula (A2-2-1) is preferably b1/c1 = 20/80 to 90/10 (mol/mol), more preferably 30/70 to 80/20 (mol/mol), even more preferably 50/50 to 70/30 (mol/mol). The proportion of b1 and c1 can be determined by $^1$H-NMR measurement.

The silicone-modified polynorbornene is preferably a silicone-modified polynorbornene represented by the following general formula (A2-2-2).

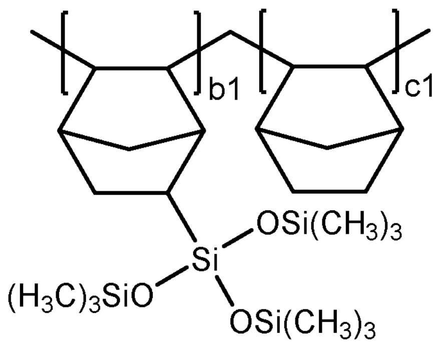
(A2-2-2)

wherein b1 and c1 are the same as above.

The silicone-modified polynorbornene represented by the general formula (A2-2-2) include a compound of, as INCI nomenclature, (norbornene/tris(trimethylsiloxy)silylnorbornene copolymer).

Commercial products of the silicone-modified polynorbornene include NBN-30-ID (isododecane solution of (norbornene/tris(trimethylsiloxy)silylnorbornene) copolymer) (by Shin-Etsu Chemical Industry Co., Ltd.).

[Silicone-Modified Pullulan (A2-3)]

The silicone-modified pullulan includes a pullulan having a silicone structure in the side chain, and specifically preferred is a silicone-modified pullulan in which at least a part of the hydrogen atoms of the OH groups in pullulan are substituted with a group represented by the following general formula (ii).

$-R^4-SiX_{a1}R^2_{3-a1}$ (ii)

wherein $R^4$ represents a single bond or a divalent organic group, and $R^2$, X and a1 are the same as above. From the viewpoint of versatility, X is preferably a trimethylsiloxyl group, and a1 is preferably 3.

In the general formula (ii), $R^4$ is preferably a divalent organic group, more preferably a divalent group represented by the following general formula (iii) or (iv), even more preferably a divalent group represented by the general formula (iv).

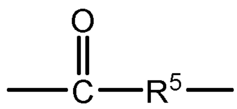 (iii)

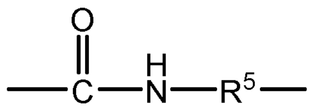 (iv)

wherein $R^5$ represents an alkylene group having 1 or more and 10 or less carbon atoms, and examples thereof include a methylene group, an ethylene group, a trimethylene group, a propylene group and a butylene group. Among these, preferred are an ethylene group, a trimethylene group and a propylene group; and more preferred are a trimethylene group and a propylene group.

Commercial products of the silicone-modified pullulan include "TSPL-30-ID" (isododecane solution of tri(trimethylsiloxy)silylpropylcarbamate pullulan), and "TSPL-30-D5" (cyclopentasiloxane solution of tri(trimethylsiloxy)silylpropylcarbamate pullulan) (all by Shin-Etsu Chemical Industry Co., Ltd.).

[Polyurea/Urethane Silicone (A2-4)]

The polyurea/urethane silicone of the component (A2-4) includes a polysiloxane/polyurea/polyurethane block terpolymer. For example, it is a dimethylpolysiloxane/urea copolymer of "polyurea-dimethicone" as INCI nomenclature.

The polymer can be produced by copolymerization of an $\alpha,\omega$-aminosilicone and a diisocyanate. Commercial products of the polyurea/urethane silicone include "Wacker-Belsil UD 60", "Wacker-Belsil UD 80", "Wacker-Belsil UD 140" and "Wacker-Belsil UD 200" (all by Wacker Corporation).

One or more can be used as the component (A). Among the above, from the viewpoint of improving film formability and durability, the component (A) preferably contains at least one selected from the group consisting of the component (A1), the component (A2-1) and the component (A2-2), more preferably at least one selected from the group consisting of the component (A1) and the component (A2-1), and even more preferably contains the component (A1), further more preferably the component (A1-2).

More specifically, from the viewpoint of improving film formability and durability, the component (A) preferably contains at least one selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, polypropylsilsesquioxane, (acrylates/polytrimethylsiloxymethacrylate) copolymer, (acrylates/dimethicone) copolymer, and (norbornene/tris(trimethylsiloxy)silylnorbornene) copolymer, more preferably contains at least one selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, polypropylsilsesquioxane, (acrylates/polytrimethylsiloxymethacrylate) copolymer, and (acrylates/dimethicone) copolymer, even more preferably contains at least one selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, and polypropylsilsesquioxane, further more preferably contains at least one selected from the group consisting of trimethylsiloxysilicate, trifluoropropyldimethyltrimethylsiloxysilicate, and (trimethylsiloxysilicate/dimethiconol) crosspolymer, further more preferably contains at least one selected from the group consisting of trimethylsiloxysilicate, and trifluoropropyldimethyltrimethylsiloxysilicate, further more preferably contains trimethylsiloxysilicate, and is further more preferably trimethylsiloxy silicate.

<Component (B): High-Molecular-Weight Organopolysiloxane>

The cosmetic composition of the present invention contains, as the component (B), a high-molecular-weight organopolysiloxane. When the cosmetic composition of the present invention is applied to a keratin substance such as skin or hair, the component (B) is considered to be eccentrically located in the outermost surface of the formed film and provide an effect of imparting a good feel, and further play a function as a surface protective layer.

In the present invention, the "high-molecular-weight organopolysiloxane" of the component (B) is an organopolysiloxane having a degree of polymerization of preferably 650 or more.

The degree of polymerization of the component (B) is, from the viewpoint of eccentric localization in the outermost surface of the formed film to give a good feel to the surface of skin or hair, more preferably 800 or more, even more preferably 1,200 or more, further more preferably 1,400 or more, further more preferably 1,900 or more, further more preferably 2,200 or more, further more preferably 2,500 or more, further more preferably 2,600 or more, further more

preferably 2,700 or more, further more preferably 2,800 or more, further more preferably 2,900 or more, further more preferably 3,000 or more, further more preferably 3,100 or more, further more preferably 3,200 or more, and is, from the viewpoint of availability, preferably 20,000 or less, more preferably 15,000 or less, even more preferably 10,000 or less, further more preferably 7,000 or less, further more preferably 5,000 or less, further more preferably 4,500 or less, further more preferably 4,300 or less, further more preferably 4,200 or less, further more preferably 4,000 or less. A specific range of the degree of polymerization of the component (B) is preferably 650 or more, more preferably 650 to 20,000, even more preferably 800 to 15,000, further more preferably 1,200 to 15,000, further more preferably 1,400 to 10,000, further more preferably 1,900 to 10,000, further more preferably 2,200 to 7,000, further more preferably 2,500 to 5,000, further more preferably 2,600 to 5,000, further more preferably 2,700 to 4,500, further more preferably 2,800 to 4,300, further more preferably 2,900 to 4,200, further more preferably 3,000 to 4,000, further more preferably 3,100 to 4,000, further more preferably 3,200 to 4,000.

More precisely, the component (B) is preferably an organopolysiloxane represented by the following general formula (1).

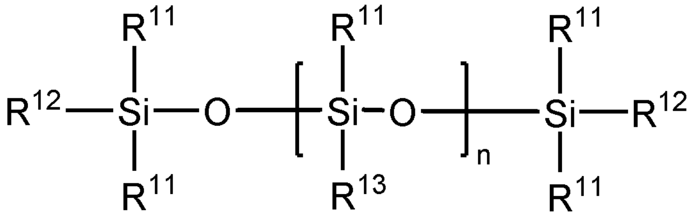

(1)

wherein $R^{11}$ each independently represents a hydrocarbon group having 1 or more and 6 or less carbon atoms, $R^{12}$ each independently represents a hydroxy group, an alkoxy group having 1 or more and 6 or less carbon atoms, or a hydrocarbon group having 1 or more and 6 or less carbon atoms, $R^{13}$ represents a hydrocarbon group having 1 or more and 6 or less carbon atoms, or a primary to tertiary amino group-containing group, n indicates a degree of polymerization, and is a number of 650 or more, and n's $R^{13}$'s can be the same as or different from each other.

In the formula (1), the hydrocarbon group for $R^{11}$ may be any of an aliphatic group or an aromatic group, and examples thereof include an alkyl group, an alkenyl group and a phenyl group. The alkyl group and the alkenyl group may be any of linear or branched ones.

Among the above, $R^{11}$ is preferably an alkyl group having 1 or more and 6 or less carbon atoms, or a phenyl group, more preferably an alkyl group having 1 or more and 3 or less car on atoms or a phenyl group, even more preferably a methyl group or a phenyl group, further more preferably a methyl group.

In the general formula (1), $R^{12}$ each independently represent a hydroxy group, an alkoxy group having 1 or more and 6 or less carbon atoms, or a hydrocarbon group having 1 or more and 6 or less carbon atoms.

The alkoxy group for $R^{12}$ includes a methoxy group, an ethoxy group, a propoxy group and a butoxy group.

The hydrocarbon group for $R^{12}$ is the same as that for $R^{11}$, and is preferably an alkyl group having 1 or more and 6 or less carbon atoms, or a phenyl group, more preferably an alkyl group having 1 or more and 3 or less carbon atoms, or a phenyl group, even more preferably a methyl group or a phenyl group, further more preferably a methyl group.

$R^{12}$ is, from the viewpoint of eccentric localization in the outermost surface of the formed film to give a good feel to the surface of skin or hair, preferably a hydroxy group, an alkyl group having 1 or more and 6 or less carbon atoms, or a phenyl group, more preferably a hydroxy group, an alkyl group having 1 or more and 3 or less carbon atoms, or a phenyl group, even more preferably a hydroxy group, a methyl group or a phenyl group, further more preferably a methyl group.

In the general formula (1), $R^{13}$ represents a hydrocarbon group having 1 or more and 6 or less carbon atoms, or a primary to tertiary amino group-containing group.

The hydrocarbon group for $R^{13}$ is the same as that for $R^{11}$, and is preferably an alkyl group having 1 or more and 6 or less carbon atoms, or a phenyl group, more preferably an alkyl group having 1 or more and 3 or less carbon atoms, or a phenyl group, even more preferably a methyl group or a phenyl group, further more preferably a methyl group.

The primary to tertiary amino group-containing group (hereinafter also referred to simply as "amino group-containing group") for $R^{13}$ is preferably a group represented by $-N(R^{14})_2$, $-NR^{14}(CH_2)_qN(R^{14})_2$, or $-NR^{14}(CH_2)_qN(R^{15})CO-R^{16}$. Here, $R^{14}$ represents a hydrogen atom or a monovalent hydrocarbon group having 1 or more and 4 or less carbon atoms, and is preferably a hydrogen atom, a methyl group or an ethyl group. $R^{15}$ represents a monovalent hydrocarbon group having 1 or more and 4 or less carbon atoms, and is preferably a methyl group or an ethyl group. $R^{16}$ represents a monovalent hydrocarbon group having 1 or more and 4 or less carbon atoms. q indicates a number of 2 or more and 6 or less, and is preferably a number of 2 or more and 4 or less.

The amino group-containing group for $R^{13}$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, more preferably $-(CH_2)_3-NH_2$.

$R^{13}$ is, from the viewpoint of eccentric localization in the outermost surface of the formed film to give a good feel to the surface of skin or hair, preferably a hydrocarbon group having 1 or more and 6 or less carbon atoms, $-(CH_2)_3-NH_2$,$-(CH_2)_3-N(CH_3)_2$, $-(CH_2)_3-NH-(CH_2)_2-NH_2$, or $-(CH_2)_2-NH-(CH_2)_2-N(CH_3)_2$, more preferably an alkyl group having 1 or more and 6 or less carbon atoms, a phenyl group, or $-(CH_2)_3-NH_2$, even more preferably an alkyl group having 1 or more and 3 or less carbon atoms, a phenyl group or $-(CH_2)_3-NH_2$, further more preferably a methyl group, a phenyl group or $-(CH_2)_3-NH_2$, further more preferably a methyl group or $-(CH_2)_3-NH_2$, further more preferably a methyl group.

In the general formula (1), n indicates a degree of polymerization and is a number of 650 or more. From the viewpoint of eccentric localization in the outermost surface of the formed film to give a good feel to the surface of skin or hair, n is preferably 800 or more, more preferably 1,200 or more, even more preferably 1,400 or more, further more preferably 1,900 or more, further more preferably 2,200 or more, further more preferably 2,500 or more, further more preferably 2,600 or more, further more preferably 2,700 or more, further more preferably 2,800 or more, further more preferably 2,900 or more, further more preferably 3,000 or more, further more preferably 3,100 or more, further more preferably 3,200 or more, and is, from the viewpoint of availability, preferably 20,000 or less, more preferably 15,000 or less, even more preferably 10,000 or less, further more preferably 7,000 or less, further more preferably 5,000 or less, further more preferably 4,500 or less, further more preferably 4,300 or less, further more preferably 4,200 or less, further more preferably 4,000 or less. A specific range of n in the general formula (1) is preferably 650 to 20,000, more preferably 800 to 15,000, even more preferably 1,200 to 15,000, further more preferably 1,400 to 10,000, further more preferably 1,900 to 10,000, further more preferably 2,200 to 7.000, further more preferably 2,500 to 5,000, further more preferably 2,600 to 5,000, further more preferably 2,700 to 4,500, further more preferably 2,800 to 4,300, further more preferably 2,900 to 4,200, further more preferably 3,000 to 4,000, further more preferably 3,100 to 4,000, further more preferably 3,200 to 4,000.

The viscosity of the component (B) is, from the viewpoint of eccentric localization in the outermost surface of the formed film to give a good feel to the surface of skin or hair, preferably 5,000 $mm^2/s$ or more, more preferably 10,000 $mm^2/s$ or more, even more preferably 100,000 $mm^2/s$ or more, further more preferably 1,000,000 $mm^2/s$ or more, further more preferably 2,000,000 $mm^2/s$ or more, further more preferably 3,000,000 $mm^2/s$ or more, further more preferably 5,000,000 $mm^2/s$ or more, further more preferably 8,000,000 $mm^2/s$ or more, further more preferably 10,000,000 $mm^2/s$ or more. The uppermost limit of the viscosity or the component (B) is, though not specifically limited but from the viewpoint of compatibility with the component (A) and availability, preferably 80,000,000 $mm^2/s$ or less, more preferably 50,000,000 $mm^2/s$ or less, even more preferably 40,000,000 $mm^2/s$ or less, further more preferably 35,000,000 $mm^2/s$ or less, further more preferably 30,000,000 $mm^2/s$ or less.

A specific range of the viscosity of the component (B) is preferably 5,000 to 80,000,000 $mm^2/s$, more preferably 10,000 to 80,000,000 $mm^2/s$, even more preferably 100,000 to 80,000,000 $mm^2/s$, further more preferably 1,000,000 to 80,000,000 $mm^2/s$, further more preferably 1,000,000 to 50,000,000 $mm^2/s$, further more preferably 2,000,000 to 50,000,000 $mm^2/s$, further more preferably 3,000,000 to 40,000,000 $mm^2/s$, further more preferably 5,000,000 to 40,000,000 $mm^2/s$, further more preferably 8,000,000 to 35,000,000 $mm^2/s$, further more preferably 10,000,000 to 30,000,000 $mm^2/s$.

The viscosity is a value measured according to JIS Z8803:2011 "Method for Measurement of Viscosity of Liquid". For example, using a suitable one selected from a capillary viscometer, a falling ball viscometer, a rotational viscometer and a vibration-type viscometer at 25°C, the viscosity can be measured. In the case where the viscosity oversteps an ordinary measurement range of a viscometer, it can be determined from a diluted solution of the component (B) according to the following method.

A toluene solution of the component (B) having a concentration of 1 g/100 mL is prepared, and a specific viscosity thereof ηsp (25°C) is calculated according to the following mathematical expression (1). Next, the resultant value is introduced into the Huggins relational expression represented by the following mathematical expression (2) to give an intrinsic viscosity [η]. Further, [η] is introduced into the expression by A. Kolorlov represented by the following mathematical expression (3) to give a molecular weight M. Finally, M is introduced into the expression by A. J. Barry represented by the following mathematical expression (4) to give a viscosity η of the component (B). (For example, see Silicone Oil KF-96 Performance Test Results 4.2, by Shin-Etsu Chemical Industry Co., Ltd.).

$$\eta sp=(\eta/\eta 0)-1 \qquad (1)$$

wherein η0 represents a viscosity of toluene, and η is a viscosity of the solution.

$$\eta sp=[\eta]+K'[\eta]^2 \qquad (2)$$

wherein the Hoggins' constant K' is one described in Nakamuta, Nihon Kagakukaishi, 77588 [1956].

$$[\eta]=0.215\times10^{-4}\,M^{0.65} \qquad (3)$$

$$\log\eta=1.00+0.0123M^{0.5} \qquad (4)$$

Commercial products of the dimethylpolysiloxane used as the component (B) include KF-96H-10000cs(viscosity 10000 $mm^2/s$), KF-96H-12500cs (viscosity 12500 $mm^2/s$), KF-96H-30000cs (viscosity 30000 $mm^2/s$), KF-96H-50000cs (viscosity 50000 $mm^2/s$), KF-96H-60000cs (viscosity 60000 $mm^2/s$), KF-96H-100000cs (viscosity 100000 $mm^2/s$), KF-96H-300000cs (viscosity 300000 $mm^2/s$), KF-96H-500000cs (viscosity 500000 $mm^2/s$), KF-96H-1000000cs (viscosity 1000000 $mm^2/s$), X-25-5686 (viscosity 3000000 $mm^2/s$, 30 mass%-isododecane solution), X-25-9074 (viscosity 30000000 $mm^2/s$, 30 mass%-isododecane solution) all by Shin-Etsu Chemical Industry Co., Ltd.), TSF451-100MA (viscosity 1000000 $mm^2/s$), TSE200A, Silsoft B3020 (viscosity 20000000 $mm^2/s$, 20 mass% isododecane solution) (all by Momentive performance Materials Corporation), and SH200-100,000cs (viscosity 100000 $mm^2/s$), SH200-1,000,000cs (viscosity 1000000 $mm^2/s$) (all by Dow Toray Corporation).

Commercial products of the aminopropylmethylpolysiloxane used as the component (B) include KF-8017 (10 mass%-low-viscosity dimethylpolysiloxane solution), KF-8018 (10 mass%-cyclopentasiloxane solution), and KF-8020 (20 mass%-low-viscosity dimethylpolysiloxane solution) (all by Shin-Etsu Chemical Industry Co., Ltd.).

Commercial products of the dimethicol used as the component (B) include X-21-5613 (20 mass%-low-viscosity dimethylpolysiloxane solution), X-21-5666 (30 mass%-cyclopentasiloxane solution), X-21-5847, and X-21-5849 (all by Shin-Etsu Chemical Industry Co., Ltd.).

The component (B) is, from the viewpoint of eccentric localization in the outermost surface of the formed film to give a good feel to the surface of skin or hair,, preferably at least one selected from the group consisting of dimethylpolysiloxane, methylphenylpolysiloxane, aminopropylmethylpolysiloxane and dimethiconol, of which the degree of polymerization falls within the above-mentioned range, more preferably dimethylpolysiloxane of which the degree of polymerization falls within the range.

<Component (C): organopolysiloxane having polyalkylene oxide moiety and cationic group, other than the component (A) and the component (B)>

The cosmetic composition of the present invention contains, as the component (C), an organopolysiloxane having a polyalkylene oxide moiety and a cationic group other than the component (A) and the component (B). Since the component (C) has high adsorbability to keratin substances such as skin or hairs, it is considered that the component can be eccentrically located on the side of a keratin substance in the formed film to make the surface thereof firmly adhere to the surfaces of keratin substances, therefore providing an effect of preventing the film from peeling away.

The cationic group that the component (C) has includes a cation group or a group capable of being ionized to be a cation group. Specifically, the group includes a primary amino group, a secondary amino group, a tertiary amino group, and a quaternary ammonium group, and is, from the viewpoint of adsorbability to the surfaces of skin or hairs, preferably at least one selected from the group consisting of a primary amino group, a secondary amino group and a tertiary amino group.

The carbon number of the alkylene oxide to constitute the polyalkylene oxide moiety of the component (C) may be 1 or more, and is, from the viewpoint of availability, preferably 2 or more, and 6 or less, more preferably 4 or less, even more preferably 3 or less. A specific range of the carbon number of the alkylene oxide to constitute the polyalkylene oxide moiety of the component (C) is preferably 1 to 6, more preferably 2 to 6, even more preferably 2 to 4, further more preferably 2 to 3, further more preferably 2.

One or more kinds of alkylene oxides may constitute the polyalkylene oxide moiety.

Specific examples of the alkylene oxide include at least one selected from the group consisting of ethylene oxide, propylene oxide, trimethylene oxide, butylene oxide, tetramethylene oxide, pentamethylene oxide, and hexamethylene oxide. Among these, from the viewpoint of availability, the alkylene oxide to constitute the polyalkylene oxide moiety is preferably at least one selected from the group consisting of ethylene oxide, propylene oxide, trimethylene oxide, butylene oxide and tetramethylene oxide, more preferably at least one selected from the group consisting of ethylene oxide, propylene oxide and trimethylene oxide, even more preferably at least one selected from the group consisting of ethylene oxide and propylene oxide, and is, from the viewpoint of making the component (E) eccentrically localized on the keratin substance side in the formed film, further more preferably ethylene oxide.

The average addition molar number of the alkylene oxide in the polyalkylene oxide moiety of the component (C) is, though not specifically limited thereto, preferably 2 or more, more preferably 4 or more, even more preferably 10

or more. From the viewpoint of availability, it is preferably 100 or less, more preferably 80 or less, further more preferably 50 or less. A specific range of the average addition molar number of the alkylene oxide in the polyalkylene oxide moiety of the component (C) is preferably 2 to 100, more preferably 4 to 100, even more preferably 4 to 80, further more preferably 4 to 50, further more preferably 10 to 50.

In the component (C), the polyalkylene oxide moiety and the cationic group may be in the organosiloxane main chain, or may be in the side chain part.

More specifically, the component (C) is more preferably at least one selected from the group consisting of an aminopolyether-modified silicone (C1) having a repeating unit represented by the following general formula (2) and an aminopolyether-modified silicone (C2) having a repeating unit represented by the following general formula (3).

In the following description, the aminopolyether-modified silicone (C1) having a repeating unit represented by the following general formula (2) may also be referred to as "component (C1)", and the aminopolyether-modified silicone (C2) having a repeating unit represented by the following general formula (3) may be as "component (C2)".

(Component (C1))

The component (C1) is an aminopolyether-modified silicone having a repeating unit represented by the following general formula (2).

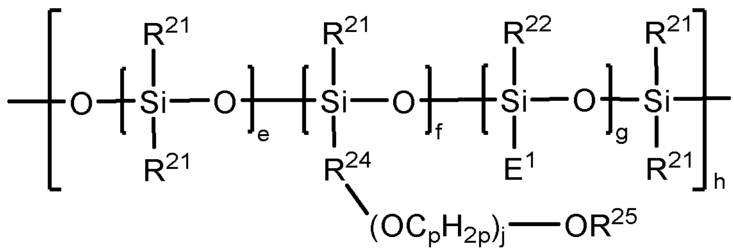

(2)

wherein $R^{21}$ each independently represents a monovalent hydrocarbon group having 1 or more and 6 or less carbon atoms,

$R^{22}$ represents any of $R^{21}$ or $E^1$, $E^1$ represents a monovalent group represented by $-R^{23}-Z^1$ (where $R^{23}$ represents a divalent hydrocarbon group having 1 or more and 6 or less carbon atoms, $Z^1$ represents a primary to tertiary amino group-containing group), $R^{24}$ represents a divalent hydrocarbon group having 1 or more and 6 or less carbon atoms, $R^{25}$ represents a hydrogen atom or a monovalent hydrocarbon group having 1 or more and 4 or less carbon atoms.

e is a number of 1 or more and 50 or less, f is a number of 1 or more and 50 or less, g is a number of 1 or more and 50 or less, h is a number of 1 or more, j is a number of 2 or more and 100 or less, p is a number of 2 or more and 10 or less. The bonding order of the parenthesized structural units is not limited, and the bonding form thereof may be in a block form or a random form. j's $(OC_pH_{2p})$s can be the same or different. Plural $R^{21}$'s, $R^{22}$'s, $R^{24}$'s, $R^{25}$'s and $E^1$'s can be the same or different.

In the general formula (2), $R^{21}$ represents a monovalent hydrocarbon group having 1 or more and 6 or less carbon atoms, and is preferably an alkyl group having 1 or more and 6 or less carbon atoms, or a phenyl group, more preferably a methyl group or an ethyl group, even more preferably a methyl group. $R^{22}$ is any of $R^{21}$ or $E^1$, and is preferably $R^{21}$.

In the general formula (2), $E^1$ represents a monovalent group represented by $-R^{23}-Z^1$ (where $R^{23}$ represents a divalent hydrocarbon group having 1 or more and 6 or less carbon atoms, $Z^1$ represents a primary to tertiary amino group-containing group). $R^{23}$ is preferably a divalent hydrocarbon group having 2 or more and 4 or less carbon atoms, more preferably an ethylene group, a trimethylene group, a propylene group or a tetramethylene group.

$Z^1$ is a primary to tertiary amino group-containing group, and is preferably a group represented by $-N(R^{26})_2$, $-NR^{26}(CH_2)_qN(R^{26})_2$, or $-NR^{26}(CH_2)_qN(R^{27})CO-R^{28}$. Here, $R^{26}$ represents a hydrogen atom or a monovalent hydrocarbon group having 1 or more and 4 or less carbon atoms, preferably a hydrogen atom, a methyl group or an ethyl group. $R^{27}$ represents a monovalent hydrocarbon group having 1 or more and 4 or less carbon atoms, and is preferably a methyl group or an ethyl group. $R^{28}$ represents a monovalent hydrocarbon group having 1 or more and 4 or less carbon atoms. q is a number of 2 or more and 6 or less, and is preferably a number of 2 or more and 4 or less.

In the general formula (2), the group $E^1$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, more preferably $-(CH_2)_3-NH_2$, or $-(CH_2)_3-NH-(CH_2)_2-NH_2$.

In the general formula (2), $R^{24}$ is a divalent hydrocarbon group having 1 or more and 6 or less carbon atoms, preferably a divalent hydrocarbon group having 2 or more and 4 or less carbon atoms, more preferably an ethylene group, a trimethylene group, a propylene group or a tetramethylene group.

$R^{25}$ is a hydrogen atom or a monovalent hydrocarbon group having 1 or more and 4 or less carbon atoms, and is preferably a methyl group or an ethyl group.

In the general formula (2), e is a number of 1 or more and 50 or less, f is a number of 1 or more and 50 or less, g is a number of 1 or more and 50 or less, h is a number of 1 or more, j is a number of 2 or more and 100 or less. p is a number of 2 or more and 10 or less, preferably 2 or more and 6 or less, more preferably 2 or more and 4 or less.

In particular, the component (C1) is more preferably one represented by the following general formula (2-1).

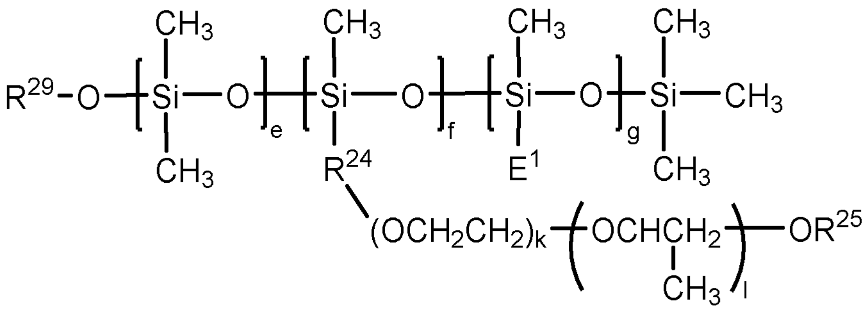

(2-1)

In the formula (2-1), $E^1$, $R^{24}$, $R^{25}$, e, f, and g are the same as above, $R^{29}$ represents a monovalent hydrocarbon group having 1 or more and 4 or less carbon atoms, or a trimethylsilyl group, k is a number of 1 or more and 50 or less, 1 is a number of 0 or more and 50 or less, and is preferably 1 or more and 50 or less.

$R^{29}$ is preferably a methyl group, an ethyl group or a trimethylsilyl group, more preferably a trimethylsilyl group.

As the component (C1), one or more can be used either singly or as combined.

As the component (C1), commercially-available aminopolyether-modified silicones can be used. Examples thereof include ABIL Soft AF10 (aminopolyether-modified silicone represented by the general formula (2-1) (methoxy PEG/PPG-7/3 aminopropyl dimethicone)) (by Evonik Corporation).

(Component (C2))

The component (C2) is an aminopolyether-modified silicone having a repeating unit represented by the following general formula (3).

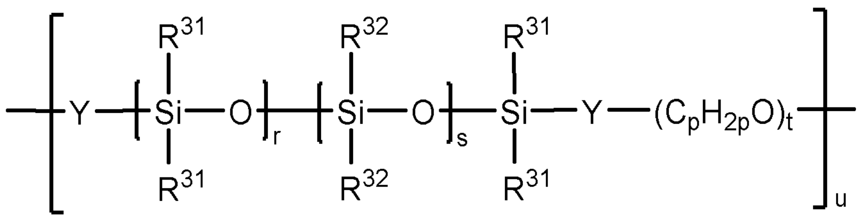

(3)

In the formula (3), $R^{31}$ represents a monovalent hydrocarbon group having 1 or more and 6 or less carbon atoms, $R^{32}$ represents any of $R^{31}$ or $E^2$, $E^2$ represents a monovalent group represented by $-R^{33}-Z^2$ (where $R^{33}$ represents a single bond, or a divalent hydrocarbon group having 1 or more and 20 or less carbon atoms, $Z^2$ represents a primary to tertiary amino group-containing group), Y represents a single bond, or a divalent group having 1 or more and 12 or less carbon atoms. In the case where all $R^{32}$'s are $R^{31}$'s, at least one Y is an amino group-containing divalent group. In the case where all Y's are divalent groups not containing an amino group, at least one $R^{32}$ is $E^2$.

r is a number of 1 or more, s is a number of 1 or more, t is a number of 2 or more and 100 or less, u is a number of 1 or more. p is the same as above, and is a number of 2 or more and 10 or less. The bonding order of the parenthesized structural units is not limited, and the bonding form thereof may be in a block form or a random form. t's $(C_pH_{2p}O)$s can be the same or different. Plural $R^{31}$'s, $R^{32}$'s, $E^2$'s and Y's can be the same or different.

In the general formula (3), $R^{31}$ represents a monovalent hydrocarbon group having 1 or more and 6 or less carbon atoms, and is independently preferably an alkyl group having 1 or more and 6 or less carbon atoms, or a phenyl group, more preferably a methyl group or an ethyl group, even more preferably a methyl group.

In the general formula (3), $R^{32}$ is any of $R^{31}$ or $E^2$. $E^2$ represents a monovalent group represented by $-R^{33}-Z^2$ (where $R^{33}$ represents a single bond, or a divalent hydrocarbon group having 1 or more and 20 or less carbon atoms).

$R^{33}$ is preferably a divalent hydrocarbon group having 1 or more and 20 or less carbon atoms, more preferably an alkylene group having 1 or more and 20 or less carbon atoms, even more preferably a linear or branched alkylene group having 1 or more and 6 or less carbon atoms, further more preferably a methylene group, an ethylene group, a

trimethylene group, a propylene group, a tetramethylene group, or a hexamethylene group, further more preferably a trimethylene group or a propylene group.

$Z^3$ is a primary to tertiary amino group-containing group, and is preferably an amino group-containing group represented by $-N(R^{34})_2$, $-NR^{34}(CH_2)_qN(R^{34})_2$, or $-NR^{34}(CH_2)_qN(R^{35})CO-R^{36}$. Here, $R^{34}$ and $R^{35}$ each independently represent a hydrogen atom or an alkyl group having 1 or more and 3 or less carbon atoms, preferably a hydrogen atom or a methyl group. $R^{36}$ represents an alkyl group having 1 or more and 3 or less carbon atoms, q is a number of 1 or more and 6 or less, and is preferably a number of 2 or more and 4 or less.

In the general formula (3), the group $E^2$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, more preferably $-(CH_2)_3-NH_2$, or $-(CH_2)_3-NH-(CH_2)_2-NH_2$.

In the general formula (3), Y is a single bond, or a divalent group having 1 or more and 12 or less carbon atoms. The divalent group having 1 or more and 12 or less carbon atoms is preferably an alkylene group having 1 or more and 6 or less carbon atoms, an alkyleneoxy group having 1 or more and 6 or less carbon atoms, or a divalent group represented by $-R^{37}-O-CH_2-CH(OH)-CH_2-N(R^{38})-R^{39}-O-$ of an amino group-containing divalent group. Here, $R^{37}$ and $R^{39}$ each independently represent an alkylene group having 1 or more and 6 or less carbon atoms, preferably an alkylene group having 2 or more and 4 or less carbon atoms, more preferably a propylene group. $R^{38}$ represents a hydrogen atom or an alkyl group having 1 or more and 3 or less carbon atoms.

In the general formula (3), the alkylene group having 1 or more and 6 or less carbon atoms and the alkylene group in the alkyleneoxy group having 1 or more and 6 or less carbon atoms for Y are preferably an ethylene group, a propylene group, a trimethylene group, an n-butylene group (tetramethylene group) or an isobutylene group, more preferably an n-butylene group or an isobutylene group. The isobutylene group as referred to herein includes $-CH(CH_3)CH_2CH_2-$,$-CH_2CH(CH_3)CH_2-$, and $-CH_2CH_2CH(CH_3)-$.

In the general formula (3), r is a number of 1 or more, s is a number of 1 or more, t is a number of 2 or more and 100 or less, u is a number of 1 or more. r is preferably a number of 1 or more and 1000 or less, more preferably a number of 2 or more and 200 or less. s is preferably a number of 1 or more and 100 or less, t is preferably a number of 4 or more and 80 or less, more preferably 10 or more and 50 or less, u is preferably a number of 1 or more and 300 or less, more preferably a number of 1 or more and 150 or less.

In the general formula (3), p is a number of 2 or more and 10 or less, preferably 2 or more and 6 or less, more preferably 2 or more and 4 or less.

The component (C2) is more preferably at least one selected from the group consisting of an aminopolyether-modified silicone having a structure represented by the following general formula (3-1) and an aminopolyether-modified silicone having a structure represented by the following general formula (3-2). Even more preferably, the component is at least one selected from the group consisting of an aminopolyether-modified silicone of a structure represented by the following general formula (3-1) and an aminopolyether-modified silicone of a structure represented by the following general formula (3-2), further more preferably an aminopolyether-modified silicone of a structure represented by the following general formula (3-1).

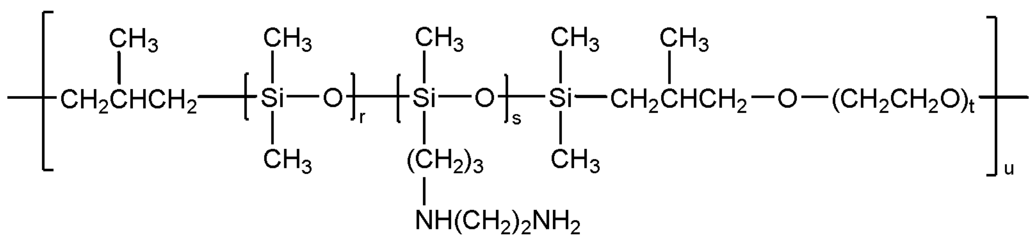

(3-1)

In the formula (3-1), r, s, t and u are the same as above.

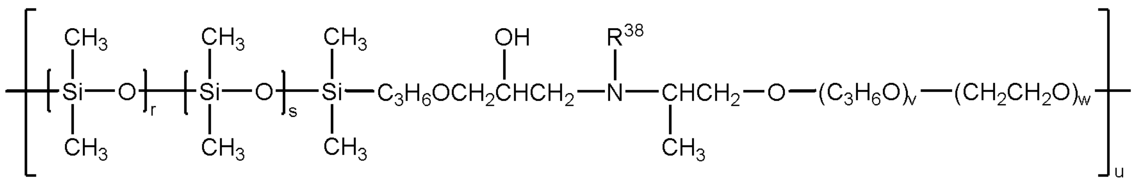

(3-2)

In the formula (3-2), $R^{38}$, r, s and u are the same as above, v is a number of 0 or more and 50 or less, preferably 2 or more and 50 or less. w is a number of 2 or more and 100 or less. v+w is a number of 2 or more and 100 or less, preferably 4 or more and 80 or less, more preferably 10 or more and 50 or less.

As the component (C2), one or more can be used either singly or as combined.

As the component (C2), commercially-available aminopolyether-modified silicones can be used. Examples thereof include, as the aminopolyether-modified silicone having a structure represented by the general formula (3-1), DOWSIL SS-3588 Fluid ((bisisobutyl-PEG-15/amodimethicone) copolymer), DOWSIL SILSTYLE 104 ((bisisobutyl-PEG-14/amodimethicone) copolymer), DOWSIL SILSTYLE 201 ((bisisobutyl-PEG-14/amodimethicone) copolymer), DOWSIL SILSTYLE 401((bisisobutyl PEG/PPG-20/35/amodimethicone) copolymer) (all by Dow Toray Corporation), and include, as the aminopolyether-modified silicone having a structure represented by the general formula (3-2), Silsoft A+ (PEG-40/PPG-8 methylaminopropyl/hydroxypropyldimethicone copolymer) (by Momentive Performance Materials Corporation).

As the component (C), one or more of the above can be used either singly or as combined. In particular, from the viewpoint of adsorbability to the surfaces of keratin substances such as skin or hairs, the component (C) is preferably an aminopolyether-modified silicone (C2) having a repeating unit represented by the above general formula (3), more preferably at least one selected from the group consisting of an aminopolyether-modified silicone having a structure represented by the above general formula (3-1) and an aminopolyether-modified silicone having a structure of the above general formula (3-2), even more preferably an aminopolyether-modified silicone having a structure of the above general formula (3-1), further more preferably a aminopolyether-modified silicone of a structure of the above general formula (3-1).

<Content>

The content of the component (A) in the cosmetic composition is, from the viewpoint of improving film formability, durability to friction and sustainability of various effects after washing, preferably 0.1% by mass or more, more preferably 0.5% by mass or more, even more preferably 1% by mass or more, further more preferably 2% by mass or more, further more preferably 2.5% by mass or more, and is, from the viewpoint of imparting a good feel to the surfaces of keratin substances such as skin or hairs, and from the viewpoint of improving durability to friction, preferably 25% by mass or less, more preferably 20% by mass or less, even more preferably 15% by mass or less. A specific range of the content of the component (A) in the cosmetic composition of the present invention is preferably 0.1 to 25% by mass, more preferably 0.1 to 20% by mass, even more preferably 0.5 to 20% by mass, further more preferably 1 to 20% by mass, further more preferably 2 to 15% by mass, further more preferably 2.5 to 15% by mass.

The content of the component (B) in the cosmetic composition is, from the viewpoint of eccentric localization in the outermost surface of the formed film to give a good feel to the surfaces of keratin substances such as skin or hairs, and from the viewpoint of improving durability to friction and sustainability of various effects after washing, preferably 0.1% by mass or more, more preferably 0.5% by mass or more, even more preferably 0.8% by mass or more, further more preferably 1% by mass or more, and is, from the viewpoint of improving durability to friction, preferably 25% by mass or less, more preferably 15% by mass or less, even more preferably 12% by mass or less, further more preferably 10% by mass or less, further more preferably 8% by mass or less. A specific range of the content of the component (B) in the cosmetic composition of the present invention is preferably 0.1 to 25% by mass, more preferably 0.5 to 15% by mass, even more preferably 0.5 to 12% by mass, further more preferably 0.8 to 10% by mass, further more preferably 1 to 10% by mass, further more preferably 1 to 8% by mass.

The content of the component (C) in the cosmetic composition of the present invention is, from the viewpoint of adsorbability to the surfaces of keratin substances such as skin or hairs, and from the viewpoint of imparting a good feel to the surfaces of keratin substances such as skin or hairs, preferably 0.01% by mass or more, more preferably 0.1% by mass or more, even more preferably 0.5% by mass or more, further more preferably 0.6% by mass or more, further more preferably 0.7% by mass or more, and is preferably 25% by mass or less, more preferably 15% by mass or less, even more preferably 12% by mass or less, further more preferably 10% by mass or less, further more preferably 5% by mass or less. A specific range of the content of the component (C) in the cosmetic composition of the present invention is preferably 0.01 to 25% by mass, more preferably 0.1 to 15% by mass, even more preferably 0.5 to 12% by mass, further more preferably 0.5 to 10% by mass, further more preferably 0.6 to 10% by mass, further more preferably 0.7 to 10% by mass, further more preferably 0.7 to 5% by mass, further more preferably 0.5 to 5% by mass.

The total content of the components (A) to (C) in the cosmetic composition of the present invention is, from the viewpoint of durability to friction and sustainability of various effects after washing, preferably 0.1% by mass or more, more preferably 0.5% by mass or more, even more preferably 1% by mass or more, further more preferably 2% by mass or more, further more preferably 3% by mass or more, further more preferably 4% by mass or more, further more preferably 5% by mass or more, and is, from the viewpoint of imparting a good feel to keratin substances in any of a dry state and a wet state, preferably 50% by mass or less, more preferably 36% by mass or less, even more preferably 25% by mass or less, further more preferably 22% by mass or less. A specific range of the total content of the component (A) to (C) in the cosmetic composition of the present invention is preferably 0.1 to 50% by mass, more preferably 0.5 to 36% by mass, even more preferably 1 to 25% by mass, further more preferably 2 to 25% by mass, further more preferably 3 to 25% by mass, further more preferably 4 to 25% by mass, further more preferably 4 to 22% by mass, further more preferably 5 to 22% by mass.

A ratio of the content by mass of the component (A) to the total content by mass of the component (A) and the component (B) in the cosmetic composition of the present invention, [(A)/((A)+(B))] is, from the viewpoint of imparting a good feel to keratin substances in any of a dry state and a wet state, and from the viewpoint of durability to friction and sustainability of various effects after washing, preferably 10% or more, more preferably 20% or more, even more preferably 25% or more, further more preferably 30% or more, further more preferably 35% or more, and is preferably 99% or less, more preferably 95% or less, even more preferably 90% or less, further more preferably 88% or less. A specific range of the ratio of the content by mass of the component (A) to the total content by mass of the component (A) and the component (B) in the cosmetic composition of the present invention, [(A)/((A)+(B))] is preferably 10 to 99%, more preferably 20 to 95%, even more preferably 20 to 90%, further more preferably 25 to 90%, further more preferably 30 to 90%, further more preferably 35 to 90%, further more preferably 35 to 88%.

A ratio of the content by mass of the component (C) to the total content by mass of the components (A) to (C) in the cosmetic composition of the present invention, [(C)/((A)+(B)+(C))] is, from the viewpoint of imparting a good feel to keratin substances in any of a dry state and a wet state, preferably 0.1% or more, more preferably 1% or more, even more preferably 2% or more, further more preferably 5% or more, further more preferably 7% or more, further more preferably 9% or more, and is, from the viewpoint of durability to friction and sustainability of various effects after washing, preferably 75% or less, more preferably 50% or less, even more preferably 35% or less, further more preferably 25% or less. A specific range of the ratio of the content by mass of the component (C) to the total content by mass of the components (A) to (C) in the cosmetic composition of the present invention, [(C)/((A)+(B)+(C))] is preferably 0.1 to 75%, more preferably 1 to 75%, even more preferably 2 to 75%, further more preferably 5 to 75%, further more preferably 5 to 50%, further more preferably 7 to 35%, further more preferably 7 to 25%, further more preferably 9 to 25%.

<Component (D): functional powder>

The cosmetic composition of the present invention can further contain a functional powder as a component (D), depending on the product form thereof.

In the present invention, the functional powder means a powder capable of providing various characteristics such as coloring performance, concealing performance, gloss, UV scattering, and feel controlling. In the case where the cosmetic composition of the present invention is a sunscreen cosmetic material, preferably, a UV scattering agent is incorporated therein as the component (D), from the viewpoint of providing a desired sunscreen effect. In the case where the cosmetic composition of the present invention is a makeup cosmetic composition or a hair dye composition, preferably, a pigment is incorporated therein as the component (D), from the viewpoint of providing a desired color tone.

As the UV scattering agent, preferably usable is at least one metal oxide powder selected from the group consisting of zinc oxide, titanium oxide and cerium oxide. An average particle size of the metal oxide powder is, from the viewpoint of UV protective effect, preferably 10 to 500 nm, more preferably 12 to 100 nm, even more preferably 15 to 50 nm. The average particle size can be measured according to a laser diffraction/scattering method.

The pigment may be any pigment generally used in makeup cosmetic materials and hair dye compositions, and examples thereof include a white inorganic pigment such as titanium oxide, zinc oxide, cerium oxide and barium sulfate; a colored inorganic pigment such as yellow iron oxide, black iron oxide, red iron oxide, carbon black, chromium oxide, chromium hydroxide, Prussian blue and ultramarine blue; a luster powder such as titanium oxide-coated mica, titanium oxide-coated bismuth oxychloride, iron oxide-coated mica titanium, iron oxide mica, Prussian blue-processed mica titanium, carmine-processed mica titanium, bismuth oxychloride, and fish scale guanine; an organic pigment such as Red No. 201, Red No. 202, Red No. 205, Red No. 226, Red No. 228, Orange No. 203, Orange No. 204, Blue No. 404, and Yellow No. 401; a chelate pigment such as a zirconium, barium or aluminum chelate of Red No. 3, Red No. 104, Red No. 106, Orange No. 205, Yellow No. 4, Yellow No. 5, Green No. 3 or Blue No. 1; and a composite pigment such as fine particle titanium oxide-coated mica titanium, fine particle zinc oxide-coated mica titanium, barium sulfate-coated mica titanium, titanium oxide-containing silicon dioxide and zinc oxide-containing silicon dioxide. One or more of these can be used either singly or as combined.

Those prepared by coating the surfaces of these functional powders with various surface treatment agents are also usable. The surface treatment agent is not specifically limited. Various surface treatments can be applied to the powders, and examples thereof include fluorine compound treatment, silicone treatment, silicone resin treatment, pendant treatment, silane coupling agent treatment, titanium coupling agent treatment, oil treatment, metal soap treatment, N-acylated lysine treatment, polyethylene glycol treatment, PVA treatment, polyacrylic acid treatment, hyaluronic acid treatment, alginic acid treatment, inorganic compound treatment, plasma treatment and mechanochemical treatment.

In the case where the cosmetic composition of the present invention contains the component (D), the content thereof is, from the viewpoint of providing desired performance, preferably 0.01% by mass or more in the cosmetic composition, more preferably 0.1% by mass or more, even more preferably 0.2% by mass or more, further more preferably 0.3% by mass or more, and is, from the viewpoint of dispersibility in the cosmetic composition and economic efficiency, and from the viewpoint of maintaining a good eel, preferably 50% by mass or less, more preferably 30% by mass or

less. A specific range of the content of the component (D) in the cosmetic composition of the present invention is preferably 0.01 to 50% by mass, more preferably 0.1 to 50% by mass, even more preferably 0.2 to 30% by mass, further more preferably 0.3 to 30% by mass.

<Component (E): solvent>

From the viewpoint of dissolving or dispersing the components (A) to (C) and other components, and from the viewpoint of controlling the viscosity to be easily applicable to keratin substances such as skin or hair, the cosmetic composition of the present invention can further contain a solvent as a component (E).

The solvent is, from the viewpoint of easy handleability, preferably a liquid organic solvent, including an alcohol solvent, an ether solvent, a ketone solvent, an ester solvent, a hydrocarbon solvent and a silicone solvent, and these can be appropriately selected depending on the formulation form. Among these, from the viewpoint of bettering sense of use after drying (from the viewpoint of reducing stickiness), a volatile solvent is preferably contained.

Among volatile solvents, the alcohol solvent includes ethanol, 1-propanol, 2-propanol, 1-butanol, 2-butanol and benzyl alcohol; the ether solvent includes diethyl ether, and tetrahydrofuran; the ketone solvent includes acetone, and methyl ethyl ketone; the ester solvent includes methyl acetate, ethyl acetate, butyl acetate, and isobutyl acetate; the hydrocarbon solvent includes light liquid isoparaffin (containing, as a main component, isoparaffin having 8 to 16 carbon atoms), pentane, isopentane, hexane, isohexene, heptane, isoheptane, decane, isodecane, dodecane, isododecane, tetradecane, isotetradecane, tridecane, and isotridecane; the silicone solvent includes a cyclic silicone such as decamethylcyclopentasiloxane, dimethylpolysiloxane having a viscosity at 25°C of 10 $mm^2/s$ or less, alkyltrimethicone such as methyltrimethicone, and methylphenylpolysiloxane having a viscosity at 25°C of 20 $mm^2/s$ or less. One or more of these can be used.

From solubility of the components (A) to (C) therein, the component (E) preferably contains at least one selected from the group consisting of dimethylpolysiloxane having a viscosity at 25°C of 10 $mm^2/s$ or less, methyltrimethicone, methylphenylpolysiloxane having a viscosity at 25°C of 20 $mm^2/s$ or less, pentane, isopentane, hexane, isohexene, heptane, isoheptane, decane, isodecane, dodecane, isododecane, tridecane, isotridecane, tetradecane, isotetradecane and light liquid isoparaffin, more preferably at least one selected from the group consisting of dimethylpolysiloxane having a viscosity at 25°C of 5 $mm^2/s$ or less, methyltrimethicone, isodecane, isododecane, isotetradecane and light liquid isoparaffin, even more preferably at least one selected from the group consisting of isodecane, isododecane, isotetradecane and light liquid isoparaffin. The viscosity is measured according to the above-mentioned measurement method.

From the viewpoint of maintaining good solubility of the components (A) to (C) therein, and from the viewpoint of controlling speed for film formation, the component (E) preferably contains a volatile alcohol solvent and at least one of a volatile hydrocarbon solvent or a volatile silicone solvent, more preferably contains at least one selected from the group consisting of ethanol, 1-propanol, 2-propanol, 1-butanol and 2-butanol, and at least one selected from the group consisting of dimethylpolysiloxane having a viscosity at 25°C of 10 $mm^2/s$ or less, methyltrimethicone, methylphenylpolysiloxane having a viscosity at 25°C of 20 $mm^2/s$ or less, pentane, isopentane, hexane, isohexene, heptane, isoheptane, decane, isodecane, dodecane, isododecane, tridecane, isotridecane, tetradecane, isotetradecane, and light liquid isoparaffin, even more preferably contains ethanol and at least one selected from the group consisting of dimethylpolysiloxane having a viscosity at 25°C of 5 $mm^2/s$ or less, methyltrimethicone, isodecane, isododecane, isotetradecane and light liquid isoparaffin, further more preferably contains ethanol and at least one selected from the group consisting of isodecane, isododecane, isotetradecane and light liquid isoparaffin.

In the case where the cosmetic composition of the present invention contains the component (E), the content thereof is, from the viewpoint of dissolving or dispersing the components (A) to (C) and other components, preferably 1% by mass or more in the cosmetic composition, more preferably 5% by mass or more, even more preferably 10% by mass or more, and is, from the viewpoint of controlling the viscosity of the cosmetic composition to be easily applicable to skin or hair, preferably 99% by mass or less, more preferably 95% by mass or less, further more preferably 90% by mass or less. A specific range of the content of the component (E) in the cosmetic composition of the present invention is preferably 1 to 99% by mass, more preferably 5 to 99% by mass, even more preferably 10 to 99% by mass, further more preferably 10 to 95% by mass, further more preferably 10 to 90% by mass.

The cosmetic composition of the present invention can contain components generally used in cosmetic compositions, for example, water, an oily agent, an antioxidant, a fragrance, a pigment, a dye, a preservative, a thickener, a pH regulator, a blood circulation promoter, a cooling sensation agent, an antiperspirant, a bactericide, a skin activator, a moisturizer and a refrigerant, in addition to the above-mentioned components.

The cosmetic composition of the present invention can be produced according to an ordinary method.

From the viewpoint of improving durability to friction, and sustainability of various effects after washing, the content of a solid oil in the cosmetic composition of the present invention is preferably small. The solid oil is an oil that is solid at 25°C, and includes a paraffin wax such as a solid paraffin, a polyolefin wax such as polyethylene wax, and beeswax. The content is preferably less than 50% by mass in the cosmetic composition, more preferably less than 20%

by mass, even more preferably less than 10% by mass, further more preferably less than 5% by mass, further more preferably less than 1% by mass.

In the cosmetic composition of the present invention, the content of a nonvolatile liquid oily agent except the component (B) and the component (C) is preferably smaller, from the viewpoint of improving durability to friction, and sustainability of various effects after washing. The nonvolatile liquid oily agent is an oily agent that has a boiling point of higher than 260°C under normal pressure and is liquid at 25°C, and examples thereof include a nonvolatile liquid silicone except the component (B) and the component (C); a triglyceride such as glyceryl trioctanoate, avocado oil, olive oil, sesame oil, rice bran oil, safflower oil, soybean oil, corn oil, rapeseed oil, castor oil, cotton seed oil, and mink oil; a fatty acid such as oleic acid and isostearic acid; an ester oil such as isopropyl myristate, butyl myristate, isopropyl palmitate, ethyl oleate, ethyl linoleate, isopropyl linoleate, cetyl caprylate, hexyl laurate, decyl myristate, decyl oleate, oleyl oleate, isostearyl laurate, isotridecyl myristate, isocetyl myristate, isostearyl myristate, octyl dodecyl myristate, octyl palmitate, isocetyl palmitate, isostearyl palmitate, isodecyl oleate, isopropyl isostearate, cetyl 2-ethylhexanoate, stearyl 2-ethylhexanoate, propylene glycol dicaprylate, propylene glycol dioleate, glyceryl tri-2-ethylhexanoate, glyceryl tri(caprate/caprylate), isononyl isononanoate, diisopropyl sebacate, propylene glycol isostearate, 2-ethylhexyl paramethoxycinnamate, 2-ethoxyethyl paramethoxycinnamate, isopropyl paramethoxycinnamate/diisopropylcinnamate mixture, methylbis(trimethylsiloxy)silylisopentyl trimethoxycinnamate, amyl paradimethylaminobenzoate, 2-ethylhexyl paradimethylaminobenzoate, ethylene glycol salicylate, 2-ethylhexyl salicylate, benzyl salicylate, homomenthyl salicylate, octocrylene, and dimethyldiethylbenzal malonate; and a branched or unsaturated higher alcohol such as 2-octyldodecanol, isostearyl alcohol, and oleyl alcohol. The content thereof is, in the cosmetic composition, preferably less than 50% by mass, more preferably less than 40% by mass, even more preferably less than 30% by mass, further more preferably less than 20% by mass, further more preferably less than 10% by mass.

From the viewpoint of increasing the drying speed after application, the content of a volatile cyclic silicone such as decamethylcyclopentasiloxane in the cosmetic composition of the present invention is preferably small. This is because the time for evaporation of the volatile cyclic silicone oil is longer than that of a volatile hydrocarbon oil and therefore the time to be taken for drying it after application to hair may tend to be longer. The content of the volatile cyclic silicone in the cosmetic composition is, from the viewpoint of increasing the drying speed after application, preferably less than 5% by mass, more preferably less than 2% by mass, even more preferably less than 1% by mass, further more preferably less than 0.5% by mass, further more preferably less than 0.1% by mass, further more preferably 0% by mass.

From the viewpoint of improving durability to friction and sustainability of various effects after washing, the content of a polyalcohol in the cosmetic composition of the present invention is preferably small. The polyalcohol includes a polyalcohol having a boiling point of higher than 260°C under normal pressure, and examples thereof include propylene glycol and glycerin. The content is preferably less than 5% by mass in the cosmetic composition, more preferably less than 2% by mass, even more preferably less than 1% by mass, further more preferably less than 0.5% by mass, further more preferably less than 0.1% by mass.

<Formulation Form, etc.>

The formulation form of the cosmetic composition of the present invention is not specifically limited, and depending on the product form thereof, the cosmetic composition can have various formation forms such as liquid, paste, cream, gel, foam, spray and wax. From the viewpoint of temporal stability of the components (A) to (C), the cosmetic composition of the present invention is preferably a waterless composition or in the form of an emulsion composition. Here, the waterless composition means a non-emulsion composition having a water content of 10% by mass or less, preferably 5% by mass or less, more preferably 2% by mass or less, even more preferably 1% by mass or less.

In the case where the cosmetic composition is in the form of an emulsion composition, the water content in the cosmetic composition is, from the viewpoint of stably forming an emulsion, preferably 1% by mass or more, more preferably 5% by mass or more, and is preferably 80% by mass or less, more preferably 50% by mass or less.

The cosmetic composition of the present invention includes various skin cosmetic compositions, eyebrow or eyelash makeup compositions and hair cosmetic compositions.

The skin cosmetic composition includes various skin cosmetic compositions for makeup, foundation, skincare, sunscreen, etc.

The eyebrow or eyelash makeup composition includes various eyebrow or eyelash makeup compositions such as mascara, mascara base coat, mascara topcoat, and eyebrow mascara.

The hair cosmetic composition includes a hair wash composition such as shampoo, as well as a rinse composition, a conditioner composition, a treatment composition (including non-washing type), a styling composition, a hair dye composition, and a hair tonic composition. Among these, from the viewpoint of effectiveness of the effect of the present invention, preferred are a conditioner composition, a treatment composition, a styling composition and a hair dye composition.

The above-mentioned composition is preferably a so-called leave-on preparation that is used without washing

after application to keratin substances such as skin, eyebrow, eyelash or hair.

[Cosmetic Kit]

The above-mentioned components (A) to (C) can be mixed before use to prepare the cosmetic composition of the present invention and can be applied to keratin substances such as skin or hair. For example, the present invention can also provide a cosmetic kit provided with at least two compositions, wherein the cosmetic composition obtained by mixing the compositions constituting the cosmetic kit contains the following components (A) to (C).

Namely, the cosmetic kit of the present invention is a cosmetic kit provided with at least two compositions, wherein the cosmetic composition obtained by mixing all the compositions constituting the cosmetic kit contains the following components (A) to (C).

(A) A silicone film-forming agent.
(B) A high-molecular-weight organopolysiloxane.
(C) An organopolysiloxane having a polyalkylene oxide moiety and a cationic group, other than the component (A) and the component (B).

Specifically, the present invention provides the following cosmetic kits 1 to 4, but is not limited thereto. The cosmetic kit of the present invention may be further provided with a composition not containing any of the components (A) to (C).

(Cosmetic Kit 1)

A cosmetic kit provided with the following compositions (I), (II) and (III).

(I) A composition containing (A) a silicone film-forming agent.
(II) A composition containing (B) a high-molecular-weight organop olysiloxane.
(III) A composition containing (C) an organopolysiloxane having a polyalkylene oxide moiety and a cationic group, other than the component (A) and the component (B).

(Cosmetic Kit 2)

A cosmetic kit provided with the following compositions (IV) and (V).

(IV) A composition containing (A) a silicone film-forming agent, and (B) a high-molecular-weight organopolysiloxane.
(V) A composition containing (C) an organopolysiloxane having a polyalkylene oxide moiety and a cationic group, other than the component (A) and the component (B).

(Cosmetic Kit 3)

A cosmetic kit provided with the following compositions (VI) and (VII).

(VI) A composition containing (A) a silicone film-forming agent.
(VII) A composition containing (B) a high-molecular-weight organopolysiloxane, and (C) an organopolysiloxane having a polyalkylene oxide moiety and a cationic group, other than the component (A) and the component (B).

(Cosmetic Kit 4)

A cosmetic kit provided with the following compositions (VIII) and (IV).

(VIII) A composition containing (A) a silicone film-forming agent, and (C) an organopolysiloxane having a polyalkylene oxide moiety and a cationic group, other than the component (A) and the component (B).
(IX) A composition containing (B) a high-molecular-weight organop olysiloxane.

The components (A), (B) and (C) used in the cosmetic kit and preferred embodiments thereof are the same as those described hereinabove for the cosmetic composition.

The compositions constituting the cosmetic kit can optionally contain, as needed, the functional powder (D), the solvent (E) and other optional components exemplified hereinabove for the cosmetic composition.

The concentration of each composition constituting the cosmetic kit is not specifically limited, but preferably, the content and the ratio of the components (A), (B) and (C) in the cosmetic composition obtained by mixing all the compositions constituting the cosmetic kit can fall within the range described hereinabove for the cosmetic composition.
Before use, all the compositions constituting the cosmetic kit of the present invention are mixed and used.

[Treatment Method for Keratin Substances]

From the viewpoint that the cosmetic composition of the present invention can promptly form a film excellent in rubfastness and wash resistance on the surfaces of keratin substances, the present invention also provides a treatment method for keratin substances, including a step of applying the cosmetic composition of the present invention to a keratin substance and then drying it.
The keratin substance includes skin, eyebrow, eyelash, hair and nails, and is preferably skin, eyelash, eyebrow or hair, more preferably hair. The keratin substance to which the cosmetic composition is applied may be in any of a dry state or wet state, but from the viewpoint of attaining the effects of the present invention, the cosmetic composition is preferably applied to a keratin substance in a dry state.
From the viewpoint of uniform application, and from the viewpoint of improving the uniformity of the structure of the film to be formed, it is preferable that the cosmetic composition of the present invention is temporarily compatibilized or dispersed prior to application to keratin substances, and then applied to the surfaces of keratin substances. As the temporarily compatibilizing or dispersing method, arbitrarily employable is any of a thermodynamical method of heating, a physical method of mechanically imparting shear stress, or a chemical method of adding a compatible solvent. From the viewpoint of user-friendliness, preferably, the cosmetic composition is uniformly compatibilized or dispersed by a physical method of stirring or shaking.
In the case where the cosmetic composition of the present invention is a skin cosmetic composition, or an eyebrow or eyelash cosmetic composition, preferably, the composition is applied to skin, eyebrow or eyelash and then spontaneously dried. From the viewpoint of maintaining various effects of the skin cosmetic composition, it is preferable that the composition is not washed off after drying, and is used as a leave-on preparation.
The skin cosmetic composition and the eyebrow or eyelash cosmetic composition is, after applied to skin, eyebrow or eyelash, preferably dried before being brought into contact with clothes and other articles. The drying time is not specifically limited so far as, after the skin cosmetic composition has been applied, it can substantially form a film on the surface of skin, eyebrow or eyelash, and the time can be appropriately controlled depending on the coating amount and the coating area, and preferably, the formed film is dried for 4 minutes or less, more preferably 2 minutes or less.
In the case where the cosmetic composition of the present invention is a hair cosmetic composition, from the viewpoint of promptly forming a film excellent in rubfastness and wash resistance on the surface of hair, it is preferable that the application process includes a step of applying the composition to hair and then drying it. From the viewpoint of maintaining various effects of the hair cosmetic composition, it is preferable that the composition is applied to hair in a dry state and then dried, and is used as a leave-on preparation that is not washed off after application. Drying the hair after application of the hair cosmetic composition thereto may be spontaneous drying, or the hair may be dried using a device such as a hair drier hood, a hand hair drier, or a straight iron.
In the case of using the device, preferably, the hair is dried at a temperature of 40 to 220°C from the viewpoint of suppressing thermal damages of keratin substances. More preferred is drying with a hair drier hood or a hand hair drier, and the drying temperature is preferably 40 to 110°C, more preferably 50 to 90°C.
The drying time is not specifically limited so far as a film is substantially formed on the surface of hair, and can be appropriately controlled depending on the amount and the quality of hair. For example, the time may fall within a range of 10 seconds to 120 minutes.
After drying, the hair may be brushed for unraveling.
In the treatment method of the present invention, the amount of the cosmetic composition to be applied to keratin substances is not specifically limited. In the case of a skin cosmetic composition, in general, the amount falls within a range of 0.1 to 1000 mg per $cm^2$ of skin. In the case of an eyebrow or eyelash cosmetic composition or a hair cosmetic composition, in general, the amount falls within a range of 0.005 to 1 g per gram of eyebrow, eyelash or hair.

[Hair Dyeing Method]

From the viewpoint of promptly forming a film excellent in rubfastness and wash resistance on the surfaces of hairs, the present invention further provides a hair dyeing method that includes a step of applying the hair dye composition of the present invention to hair and then drying it.
The hair dye composition of the present invention is applied to hair and then dried, and is used without washing. Drying the hair after applying the hair dye composition thereto may be spontaneous drying, or the hair may be dried with

a drier or the like.

After drying, the hair can be brushed for unraveling.

By the above-mentioned simple operation, the hair dye composition of the present invention can temporarily or semi-permanently dye hair as an out-bath treatment. In addition, the composition can give a good feel to hair in any of a dry state and a wet state, and the color film peels little by friction after hair dyeing, and can suppress secondary adhesion to clothes and pillows. In addition, after shampooing, discoloration is low and color sustainability is good, and further, even after shampooing, a good feel retains and a secondary adhesion suppressing effect can also be sustained.

[Film Forming Method and Film]

The present invention provides a method for forming a film on the surface of a keratin substance, including the following step (I) to step (III) in that order (hereinafter also simply referred to as "the method of the present invention").

Step (I): a step of temporarily compatibilizing or dispersing a composition containing the following components (A) to (C),

(A) a silicone film-forming agent,
(B) a high-molecular-weight organopolysiloxane,
(C) an organopolysiloxane having a polyalkylene oxide moiety and a cationic group, other than the component (A) and the component (B),

Step (II): a step of applying the composition prepared in the step (I) to the surface of a keratin substance,
Step (III): a step of drying the composition applied to the surface of a keratin substance in the step (II).

The present invention also provides a film to cover the surface of a keratin substance, wherein the film is a multilayer-structured film containing the components (A) to (C), having a layer containing the component (B) in the outermost surface of the film, and having a layer containing the component (C) on the interface side to the keratin substance (hereinafter also simply referred to as "the film of the present invention").

(A) a silicone film-forming agent,
(B) a high-molecular-weight organopolysiloxane,
(C) an organopolysiloxane having a polyalkylene oxide moiety and a cationic group, other than the component (A) and the component (B).

By applying the composition containing the components (A) to (C) to the surface of a keratin substance, the film of the present invention can be formed.

It is difficult for a uniform film to satisfy plural properties all at a time, and a method of forming a film that satisfies plural properties all at a time by laminating plural layers differing in properties can be investigated, but such a method of laminating layers plural times is complicated, and it is desirable to form a film having a multilayered structures in a one-step process. According to the film forming method of the present invention, a composition containing the components (A) to (C) is temporarily compatibilized or dispersed in the step (I) before use, and then in the step (II), the composition is applied to the surface of a keratin substance, and dried in the step (III). By the operation, it is considered that the components can undergo phase separation with time and can spontaneously form a film having the above-mentioned multilayered structure.

In the step (I), for the means of temporarily compatibilizing or dispersing the composition containing the components (A) to (C), a thermomechanical means of heating; a physical means of mechanically applying shear stress; or a chemical means of adding a solvent compatible or dispersible with the components (A) to (C) can be employed in any desired manner. From the viewpoint of users' convenience, preferably, the components are uniformly compatibilized or dispersed by a physical means of stirring or shaking. In the case, from the viewpoint of facilitating the physical means of stirring or shaking, preferably, a solvent having compatibility and dispersibility with the components (A) to (C) (component (E)) is previously added to the composition.

The solvent added to the composition is, from the viewpoint of promoting film formation, preferably removed from the film by drying in the step (III) after application to the surface of a keratin substance in the step (II). For example, employable is a method of preparing the composition using the above-mentioned volatile solvent as a solvent in the step (I), then drying in the step (III) after application to the surface of a keratin substance in the step (II) to thereby remove the volatile solvent from the film by vaporization.

A preferred film structure to be formed according to the method of the present invention is a multilayered structure having a concentration distribution of each component in the thickness direction formed on the surface of a

keratin substance, and is specifically a multilayered structure having at least two layers including a layer containing the component (B) in the outermost surface of the film and a layer containing the component (C) on the interface side to the keratin substance, and is, from the viewpoint of sustaining the strength and the durability of the entire formed film, preferably a multilayered structure of at least three layers having a layer containing the component (A) between a layer containing the component (B) and a layer containing the component (C).

In application of the cosmetic composition of the present indention to the surface of a keratin substance, it is considered that the component (B) is eccentrically located in the outermost surface of the formed film and can exhibit an effect of imparting a good feel to the surface of a keratin substance in any of a dry state and a wet state, and further can play a function as a surface protective layer. It is considered that the component (C) is eccentrically located on the keratin substance side and exhibits a film peeling preventing effect by firmly adhering the formed film to the surface of a keratin substance.

On the other hand, the component (A) is a hydrophobic film-forming agent, and is considered to exist in the layer containing the component (B) or the component (C) or to be eccentrically located in the intermediate part of the layer containing the component (B) and the component (C) in the formed film to thereby secure the strength and the durability of the entire formed film, and is also considered to exhibit an effect of improving durability of various effects imparted to the keratin substance, owing to the synergistic effect with the component (B) and the component (C).

The components (A), (B) and (C) constituting the film and the preferred embodiments thereof are the same as those described hereinabove for the cosmetic composition.

From the viewpoint of forming a good multilayered film, the component (A) preferably contains a trimethylsiloxysilicate, and is more preferably a trimethylsiloxysilicate. The degree of polymerization of the component (B) is preferably 650 to 20,000, more preferably 800 to 15,000, even more preferably 1,200 to 15,000, further more preferably 1,400 to 10,000, further more preferably 1,900 to 10,000, further more preferably 2,200 to 7,000, further more preferably 2,500 to 5,000, further more preferably 2,600 to 5,000, further more preferably 2,700 to 4,500, further more preferably 2,800 to 4,300, further more preferably 2,900 to 4,200, further more preferably 3,000 to 4,000, further more preferably 3,100 to 4,000, further more preferably 3,200 to 4,000. The component (C) is preferably an aminopolyether-modified silicone (C2) having a repeating unit represented by the general formula (3), more preferably at least one selected from the group consisting of an aminopolyether-modified silicone having a structure represented by the general formula (3-1) and an aminopolyether-modified silicone having a structure represented by the general formula (3-2), even more preferably an aminopolyether-modified silicone having a structure represented by the general formula (3-1), further more preferably an aminopolyether-modified silicone formed of a structure represented by the general formula (3-1).

The film of the present invention can optionally contain, as needed, the functional powder (D), the solvent (E) and other optional components exemplified hereinabove for the cosmetic composition.

In the case where the film of the present invention contains an optional component of the component (D), the content of the component (D) in the film is, from the viewpoint of sufficiently expressing the effect of the functional powder, preferably 0.01% by mass or more, more preferably 0.1% by mass or more, even more preferably 0.2% by mass or more, further more preferably 0.3% by mass or more. From the viewpoint of dispersibility in the film, the content is preferably 50% by mass or less, more preferably 30% by mass or less. A specific range of the content of the component (D) in the film is preferably 0.01 to 50% by mass, more preferably 0.1 to 50% by mass, even more preferably 0.2 to 30% by mass, further more preferably 0.3 to 30% by mass.

The total content of the components (A) to (C) in the film of the present invention is, from the viewpoint of durability to friction and sustainability of various effects after washing, preferably 50% by mass or more, more preferably 70% by mass or more, even more preferably 80% by mass or more, further more preferably 90% by mass or more, and is 100% by mass or less.

In the case where the film of the present invention contains the component (D) and others, the total content of the components (A) to (C) in the film is preferably 99.99% by mass or less, more preferably 99.9% by mass or less, even more preferably 99.8% by mass or less, further more preferably 99.7% by mass or less.

A ratio by mass of the content of the component (A) to the total content of the component (A) and the component (B) in the film of the present invention, [(A)/((A)+(B))] is, from the viewpoint of imparting a good feel to keratin substances in any of a dry state and a wet state, and from the viewpoint of durability to friction and sustainability of various effects after washing, preferably 10% or more, more preferably 20% or more, even more preferably 30% or more, further more preferably 35% or more, and is preferably 99% or less, more preferably 95% or less, even more preferably 90% or less, further more preferably 88% or less. A specific range of the ratio of the content by mass of the component (A) to the total content by mass of the component (A) and the component (B) in the film of the present invention, [(A)/((A)+(B))] is preferably 10 to 99%, more preferably 20 to 95%, even more preferably 20 to 90%, further more preferably 25 to 90%, further more preferably 30 to 90%, further more preferably 35 to 90%, further more preferably 35 to 88%.

A ratio by mass of the content of the component (C) to the total content of the components (A) to (C) in the film of the present invention, [(C)/((A)+(B)+(C))] is, from the viewpoint of imparting a good feel to keratin substances in any of a dry state and a wet state, preferably 0.1% or more, more preferably 1% or more, even more preferably 2% or more,

further more preferably 5% or more, further more preferably 7% or more, further more preferably 9% or more, and is, from the viewpoint of durability to friction and sustainability of various effects after washing, preferably 75% or less, more preferably 50% or less, even more preferably 35% or less, further more preferably 25% or less. A specific range of the ratio of the content by mass of the component (C) to the total content by mass of the components (A) to (C) in the film of the present invention, [(C)/((A)+(B)+(C))] is preferably 0.1 to 75%, more preferably 1 to 75%, even more preferably 2 to 75%, further more preferably 5 to 75%, further more preferably 5 to 50%, further more preferably 7 to 35%, further more preferably 7 to 25%, further more preferably 9 to 25%.

The thickness of the film of the present invention can appropriately vary but is, from the viewpoint of improving durability to friction and sustainability of various effects after washing, preferably 50 nm or more, more preferably 100 nm or more, even more preferably 200 nm or more. From the viewpoint of improving the feel in use in a dry state, the thickness is preferably 50 pm or less, more preferably 20 pm or less, even more preferably 10 pm or less. For measuring the thickness of the film, any known method can be appropriately selected, depending on the range of the thickness of the film. By observation with various microscopes of a cross section of a keratin substance treated with the composition of the present invention, or a cross section of the film peeled from a keratin substance, or by measurement of the film peeled from a keratin substance, or from the change in the thickness or the size of a keratin substance before and after film formation thereon, the film thickness can be determined. For example, the thickness of the film formed on the surface of hair can be calculated from the change in the hair diameter before and after film formation. After treated with the composition of the present invention, a cross section of hair can be observed with an atomic force microscope in an AMFM mode to measure the formed film, as shown in Examples.

Regarding the above-mentioned embodiments, the present invention discloses the following.

<1> A cosmetic composition containing the following components (A) to (C),

(A) a silicone film-forming agent,
(B) a high-molecular-weight organopolysiloxane,
(C) an organopolysiloxane having a polyalkylene oxide moiety and a cationic group, other than the component (A) and the component (B).

<2> A cosmetic composition containing the following components (A) to (C), wherein:
a ratio by mass of the content of the component (C) to the total content of the components (A) to (C) in the cosmetic composition, [(C)/(A)+(B)+(C)] is 7% or more and 35% or less, a ratio by mass of the content of the component (A) to the total content of the component (A) and the component (B) in the cosmetic composition, [(A)/(A)+(B)] is 20% or more and 95% or less, and the total content of the components (A) to (C) in the cosmetic composition is 1% by mass or more and 25% by mass or less,

(A) a silicone film-forming agent,
(B) a high-molecular-weight organopolysiloxane,
(C) an organopolysiloxane having a polyalkylene oxide moiety and a cationic group, other than the component (A) and the component (B).

<3> A cosmetic composition containing the following components (A) to (C), wherein:
a ratio by mass of the content of the component (C) to the total content of the components (A) to (C) in the cosmetic composition, [(C)/(A)+(B)+(C)] is 9% or more and 25% or less, a ratio by mass of the content of the component (A) to the total content of the component (A) and the component (B) in the cosmetic composition, [(A)/(A)+(B)] is 20% or more and 95% or less, and the total content of the components (A) to (C) in the cosmetic composition is 4% by mass or more and 22% by mass or less,

(A) a silicone film-forming agent,
(B) a high-molecular-weight organopolysiloxane,
(C) an organopolysiloxane having a polyalkylene oxide moiety and a cationic group, other than the component (A) and the component (B).

<4> A cosmetic composition containing the following components (A) to (C), wherein:

a ratio by mass of the content of the component (C) to the total content of the components (A) to (C) in the cosmetic composition, [(C)/((A)+(B)+(C))], is 7% or more and 35% or less, a ratio by mass of the content of the component (A) to the total content of the component (A) and the component (B) in the cosmetic composition, [(A)/(A)+(B)] is 20% or more and 95% or less, and the total content of the components (A) to (C) in the cosmetic

composition is 1% by mass or more and 25% by mass or less,

(A) 0.1 to 25% by mass of a silicone resin represented by an average formula, $(R^1)_mSiO_{(4-m)/2}$

wherein $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3,
which contains a Q unit represented by $SiO_{4/2}$ and an M unit represented by $(R^1)_3SiO_{1/2}$,

(B) 0.1 to 25% by mass of an organopolysiloxane represented by the following general formula (1),

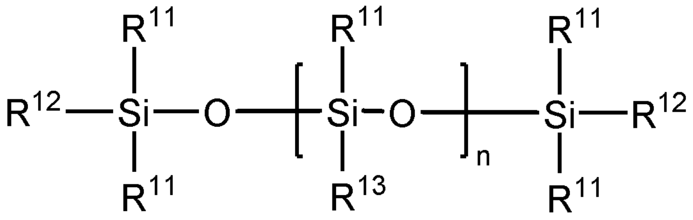 (1)

wherein $R^{11}$ each independently represents a hydrocarbon group having 1 or more and 6 or less carbon atoms, $R^{12}$ each independently represents a hydroxy group, an alkoxy group having 1 or more and 6 or less carbon atoms, or a hydrocarbon group having 1 or more and 6 or less carbon atoms, $R^{13}$ represents a hydrocarbon group having 1 or more and 6 or less carbon atoms, or a primary to tertiary amino group-containing group, n indicates a degree of polymerization, and is a number of 800 to 15,000, and n's $R^{13}$'s can be the same as or different from each other,
(C) 0.01 to 25% by mass of an organopolysiloxane having a polyalkylene oxide moiety and a cationic group other than the component (A) and the component (B), wherein the carbon number of the alkylene oxide in the polyalkylene oxide moiety is 1 to 6, the alkylene oxide average addition molar number is 2 to 100, and the cationic group is at least one selected from the group consisting of a primary amino group, a secondary amino group and a tertiary amino group.

<5> A cosmetic composition containing the following components (A) to (C), wherein:
a ratio by mass of the content of the component (C) to the total content of the components (A) to (C) in the cosmetic composition, [(C)/((A)+(B)+(C))], is 9% or more and 25% or less, a ratio by mass of the content of the component (A) to the total content of the component (A) and the component (B) in the cosmetic composition, [(A)/(A)+(B)] is 20% or more and 95% or less, and the total content of the components (A) to (C) in the cosmetic composition is 4% by mass or more and 22% by mass or less,

(A) 0.1 to 25% by mass of a silicone resin represented by an average formula, $(R^1)_mSiO_{(4-m)/2}$

wherein $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3,
which contains a Q unit represented by $SiO_{4/2}$ and an M unit represented by $(R^1)_3SiO_{1/2}$,

(B) 0.1 to 25% by mass of an organopolysiloxane represented by the following general formula (1),

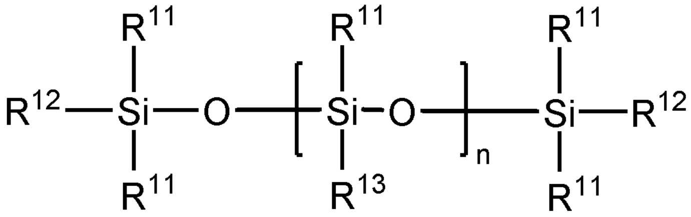 (1)

wherein $R^{11}$ each independently represents a hydrocarbon group having 1 or more and 6 or less carbon atoms, $R^{12}$ each independently represents a hydroxy group, an alkoxy group having 1 or more and 6 or less carbon atoms, or a hydrocarbon group having 1 or more and 6 or less carbon atoms, $R^{13}$ represents a hydrocarbon

group having 1 or more and 6 or less carbon atoms, or a primary to tertiary amino group-containing group, n indicates a degree of polymerization, and is a number of 800 to 15,000, and n's $R^{13}$'s can be the same as or different from each other,

(C) 0.01 to 25% by mass of an organopolysiloxane having a polyalkylene oxide moiety and a cationic group other than the component (A) and the component (B), wherein the carbon number of the alkylene oxide in the polyalkylene oxide moiety is 1 to 6, the alkylene oxide average addition molar number is 2 to 100, and the cationic group is at least one selected from the group consisting of a primary amino group, a secondary amino group and a tertiary amino group.

<6> A cosmetic composition containing the following components (A) to (C), wherein:
a ratio by mass of the content of the component (C) to the total content of the components (A) to (C) in the cosmetic composition, [(C)/((A)+(B)+(C))], is 7% or more and 35% or less, a ratio by mass of the content of the component (A) to the total content of the component (A) and the component (B) in the cosmetic composition, [(A)/(A)+(B)] is 20% or more and 95% or less, and the total content of the components (A) to (C) in the cosmetic composition is 1% by mass or more and 25% by mass or less,

(A) 0.1 to 25% by mass of a silicone resin represented by $[SiO_{4/2}]_c[(R^1)_3SiO_{1/2}]_d$ wherein c and d each are an average repeating unit number and $c>0$ and $d>0$,

(B) 0.1 to 25% by mass of an organopolysiloxane represented by the following general formula (1),

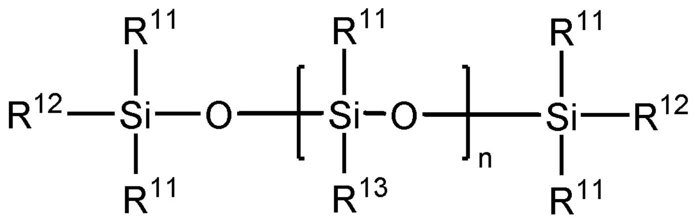
(1)

wherein $R^{11}$ each independently represents a hydrocarbon group having 1 or more and 6 or less carbon atoms, $R^{12}$ each independently represents a hydroxy group, an alkoxy group having 1 or more and 6 or less carbon atoms, or a hydrocarbon group having 1 or more and 6 or less carbon atoms, $R^{13}$ represents a hydrocarbon group having 1 or more and 6 or less carbon atoms, or a primary to tertiary amino group-containing group, n indicates a degree of polymerization, and is a number of 1,400 to 10,000, and n's $R^{13}$'s can be the same as or different from each other,

(C) 0.01 to 25% by mass of an aminopolyether-modified silicone having a repeating unit represented by the following general formula (3),

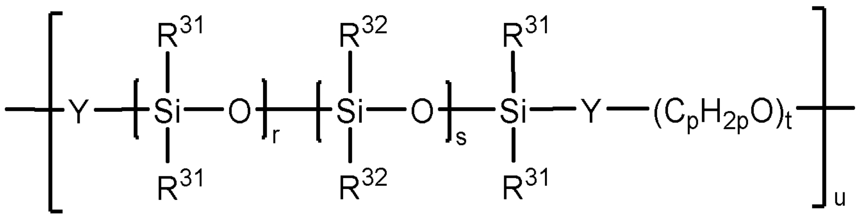
(3)

wherein $R^{31}$ represents a monovalent hydrocarbon group having 1 or more and 6 or less carbon atoms, $R^{32}$ represents any of $R^{31}$ or $E^2$, $E^2$ represents a monovalent group represented by $-R^{33}-Z^2$ (where $R^{33}$ represents a single bond, or a divalent hydrocarbon group having 1 or more and 20 or less carbon atoms, $Z^2$ represents a primary to tertiary amino group-containing group), Y represents a single bond, or a divalent group having 1 or more and 12 or less carbon atoms, in the case where all $R^{32}$'s are $R^{31}$'s, at least one Y is an amino group-containing divalent group, in the case where all Y's are divalent groups not containing an amino group, at least one $R^{32}$ is $E^2$,

r is a number of 1 or more, s is a number of 1 or more, t is a number of 2 or more and 100 or less, u is a number of 1 or more, p is is a number of 2 or more and 10 or less, the bonding order of the parenthesized structural units is not limited, and the bonding form thereof may be in a block form or a random form, t's $(C_pH_{2p}O)$s can be the same or different, plural $R^{31}$'s, $R^{32}$'s, $E^2$'s and Y's can be the same or different.

<7> A cosmetic composition containing the following components (A) to (C), wherein:

a ratio by mass of the content of the component (C) to the total content of the components (A) to (C) in the cosmetic composition, [(C)/((A)+(B)+(C))], is 9% or more and 25% or less, a ratio by mass of the content of the component (A) to the total content of the component (A) and the component (B) in the cosmetic composition, [(A)/(A)+(B)] is 20% or more and 95% or less, and the total content of the components (A) to (C) in the cosmetic composition is 4% by mass or more and 22% by mass or less,

(A) 0.1 to 25% by mass of a silicone resin represented by $[SiO_{4/2}]_c[(R^1)_3SiO_{1/2}]_d$ wherein c and d each are an average repeating unit number and c>0 and d>0,
(B) 0.1 to 25% by mass of an organopolysiloxane represented by the following general formula (1),

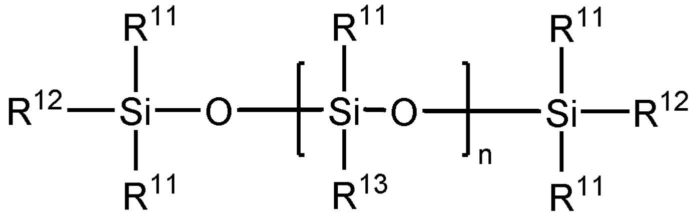 (1)

wherein $R^{11}$ each independently represents a hydrocarbon group having 1 or more and 6 or less carbon atoms, $R^{12}$ each independently represents a hydroxy group, an alkoxy group having 1 or more and 6 or less carbon atoms, or a hydrocarbon group having 1 or more and 6 or less carbon atoms, $R^{13}$ represents a hydrocarbon group having 1 or more and 6 or less carbon atoms, or a primary to tertiary amino group-containing group, n indicates a degree of polymerization, and is a number of 1,400 to 10,000, and n's $R^{13}$'s can be the same as or different from each other,
(C) 0.01 to 25% by mass of an aminopolyether-modified silicone having a repeating unit represented by the following general formula (3),

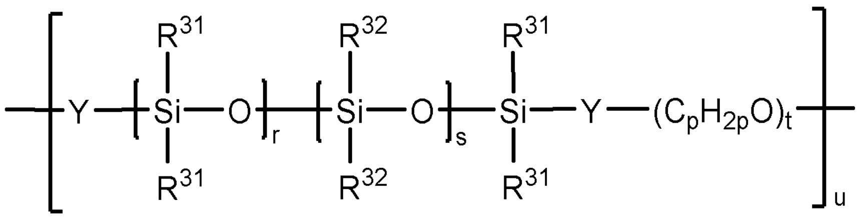 (3)

wherein $R^{31}$ represents a monovalent hydrocarbon group having 1 or more and 6 or less carbon atoms, $R^{32}$ represents any of $R^{31}$ or $E^2$, $E^2$ represents a monovalent group represented by $-R^{33}-Z^2$ (where $R^{33}$ represents a single bond, or a divalent hydrocarbon group having 1 or more and 20 or less carbon atoms, $Z^2$ represents a primary to tertiary amino group-containing group), Y represents a single bond, or a divalent group having 1 or more and 12 or less carbon atoms, in the case where all $R^{32}$'s are $R^{31}$'s, Y is an amino group-containing divalent group, in the case where all Y's are divalent groups not containing an amino group, at least one $R^{32}$ is $E^2$,
r is a number of 1 or more, s is a number of 1 or more, t is a number of 2 or more and 100 or less, u is a number of 1 or more, p is a number of 2 or more and 10 or less, the bonding order of the parenthesized structural units is not limited, and the bonding form thereof may be in a block form or a random form, t's $(C_pH_{2p}O)$s can be the same or different, plural $R^{31}$'s, $R^{32}$'s, $E^2$'s and Y's can be the same or different.

<8> The cosmetic composition according to any one of <1> to <3>, wherein the component (A) is at least one selected from the group consisting of the following components (A1) and (A2),

(A1) a silicone resin represented by an average formula, $(R^1)_mSiO_{(4-m)/2}$

wherein $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3,
which contains at least one unit selected from the group consisting of a T unit represented by $R^1SiO_{3/2}$ and a Q unit represented by $SiO_{4/2}$,

(A2) a silicone polymer containing a polysiloxane moiety and a moiety formed of a non-silicone organic chain.

<9> The cosmetic composition according to <8>, wherein the component (A1) contains at least one selected from the group consisting of the following component (A1-1) and component (A1-2),

(A1-1) a silicone resin represented by the above-mentioned average formula, containing a T unit represented by $R^1SiO_{3/2}$ and substantially not containing a Q unit represented by $SiO_{4/2}$,
(A1-2) a silicone resin represented by the above-mentioned average formula, and containing a Q unit represented by $SiO_{4/2}$ and an M unit represented by $(R^1)_3SiO_{1/2}$.

<10> The cosmetic composition according to <8> or <9>, wherein the component (A2) contains at least one selected from the group consisting of the following components (A2-1) and component (A2-4),

(A2-1) an acryl silicone polymer,
(A2-2) a silicone-modified alicyclic structure-containing polymer,
(A2-3) a silicone-modified pullulan,
(A2-4) a polyurea/urethane silicone.

<11> The cosmetic composition according to <9> or <10>, wherein the component (A1) contains the silicone resin (A1-2).
<12> The cosmetic composition according to any one of <10> or <11>, wherein the component (A2) contains the acryl silicone polymer (A2-1).
<13> The cosmetic composition according to any one of <1> to <3>, and <8> to <12>, wherein the component (A) contains at least one selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, polypropylsilsesquioxane, (acrylates/polytrimethylsiloxymethacrylate) copolymer, (acrylates/dimethicone) copolymer, and (norbornene/tris(trimethylsiloxy)silylnorbornene) copolymer, preferably contains at least one selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, polypropylsilsesquioxane, (acrylates/polytrimethylsiloxymethacrylate) copolymer, and (acrylates/dimethicone) copolymer, more preferably contains at least one selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, and polypropylsilsesquioxane, even more preferably contains at least one selected from the group consisting of trimethylsiloxysilicate, trifluoropropyldimethyltrimethylsiloxysilicate, and (trimethylsiloxysilicate/dimethiconol) crosspolymer, further more preferably contains at least one selected from the group consisting of trimethylsiloxysilicate, and trifluoropropyldimethyltrimethylsiloxysilicate, further more preferably contains trimethylsiloxysilicate, and is further more preferably trimethylsiloxysilicate.
<14> The cosmetic composition according to any one of <1> to <3>, and <8> to <13>, wherein the degree of polymerization of the component (B) is 650 or more.
<15> The cosmetic composition according to any one of <1> to <3>, and <8> to <11>, wherein the component (B) is an organopolysiloxane represented by the following general formula (1),

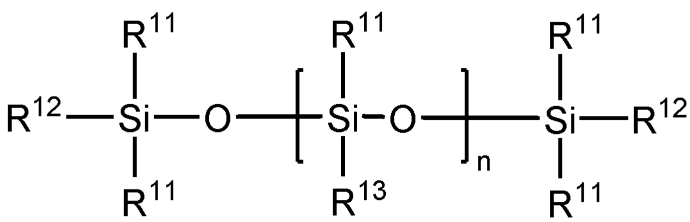
(1)

wherein $R^{11}$ each independently represents a hydrocarbon group having 1 or more and 6 or less carbon atoms, $R^{12}$ each independently represents a hydroxy group, an alkoxy group having 1 or more and 6 or less carbon atoms, or a hydrocarbon group having 1 or more and 6 or less carbon atoms, $R^{13}$ represents a hydrocarbon group having 1 or more and 6 or less carbon atoms, or a primary to tertiary amino group-containing group, n indicates a degree of polymerization, and is a number of 650 or more, and n's $R^{13}$'s can be the same as or different from each other.
<16> The cosmetic composition according to any one of <4> to <6> and <15>, wherein the degree of polymerization in the general formula (1) is preferably 650 to 20,000, more preferably 800 to 15,000, even more preferably 1,200 to 15,000, further more preferably 1,400 to 10,000, further more preferably 1,900 to 10,000, further more preferably

2,200 to 7,000, further more preferably 2,500 to 5,000, further more preferably 2,600 to 5,000, further more preferably 2,700 to 4,500, further more preferably 2,800 to 4,300, further more preferably 2,900 to 4,200, further more preferably 3,000 to 4,000, further more preferably 3,100 to 4,000, further more preferably 3,200 to 4,000.

<17> The cosmetic composition according to any one of <1> to <3> and <8> to <16>, wherein the alkylene oxide constituting the polyalkylene oxide moiety of the component (C) is at least one selected from the group consisting of ethylene oxide and propylene oxide.

<18> The cosmetic composition according to <17>, wherein the carbon number of the alkylene oxide in the polyalkylene oxide moiety of the component (C) is 1 to 6 and the alkylene oxide average addition molar number is 2 to 100, and the cationic group is at least one selected from the group consisting of a primary amino group, a secondary amino group and a tertiary amino group.

<19> The cosmetic composition according to any one of <1> to <3> and <8> to <17>, wherein the component (C) is at least one selected from the group consisting of an aminopolyether-modified silicone (C1) having a repeating unit represented by the following general formula (2) and an aminopolyether-modified silicone (C2) having a repeating unit represented by the following general formula (3),

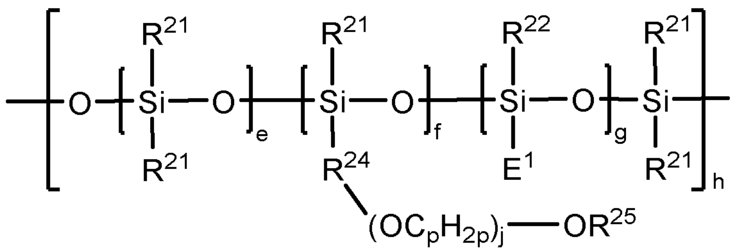

(2)

wherein $R^{21}$ each independently represents a monovalent hydrocarbon group having 1 or more and 6 or less carbon atoms, $R^{22}$ represents any of $R^{21}$ or $E^1$, $E^1$ represents a monovalent group represented by $-R^{23}-Z^1$ (where $R^{23}$ represents a divalent hydrocarbon group having 1 or more and 6 or less carbon atoms, $Z^1$ represents a primary to tertiary amino group-containing group), $R^{24}$ represents a divalent hydrocarbon group having 1 or more and 6 or less carbon atoms, $R^{25}$ represents a hydrogen atom or a monovalent hydrocarbon group having 1 or more and 4 or less carbon atoms,

e is a number of 1 or more and 50 or less, f is a number of 1 or more and 50 or less, g is a number of 1 or more and 50 or less, h is a number of 1 or more, j is a number of 2 or more and 100 or less, p is a number of 2 or more and 10 or less, the bonding order of the parenthesized structural units is not limited, and the bonding form thereof may be in a block form or a random form, j's $(OC_pH_{2p})$s can be the same or different, plural $R^{21}$'s, $R^{22}$'s, $R^{24}$'s, $R^{25}$'s and $E^1$'s can be the same or different,

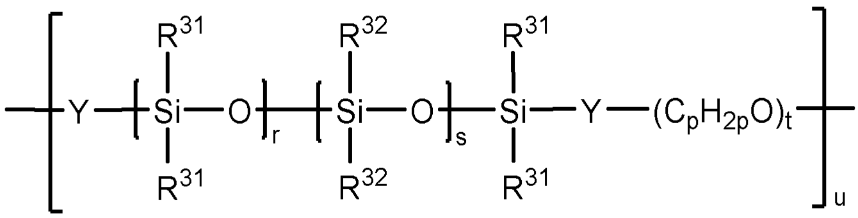

(3)

wherein $R^{31}$ represents a monovalent hydrocarbon group having 1 or more and 6 or less carbon atoms, $R^{32}$ represents any of $R^{31}$ or $E^2$, $E^2$ represents a monovalent group represented by $-R^{33}-Z^2$ (where $R^{33}$ represents a single bond, or a divalent hydrocarbon group having 1 or more and 20 or less carbon atoms, $Z^2$ represents a primary to tertiary amino group-containing group), Y represents a single bond, or a divalent group having 1 or more and 12 or less carbon atoms, in the case where all $R^{32}$'s are $R^{31}$'s, Y is an amino group-containing divalent group, in the case where all Y's are divalent groups not containing an amino group, at least one $R^{32}$ is $E^2$, r is a number of 1 or more, s is a number of 1 or more, t is a number of 2 or more and 100 or less, u is a number of 1 or more, p is a number of 2 or more and 10 or less, the bonding order of the parenthesized structural units is not limited, and the bonding form thereof may be in a block form or a random form, t's $(C_pH_{2p}O)$s can be the same or different, plural $R^{31}$'s, $R^{32}$'s, $E^2$'s and Y's can be the same or different.

<20> The cosmetic composition according to <19>, wherein the component (C) is an aminopolyether-modified silicone (C2) having a repeating unit represented by the general formula (3).

<21> The cosmetic composition according to <19> or <20>, wherein the component (C1) is an aminopolyether-modified silicone (C2) represented by the following general formula (2-1),

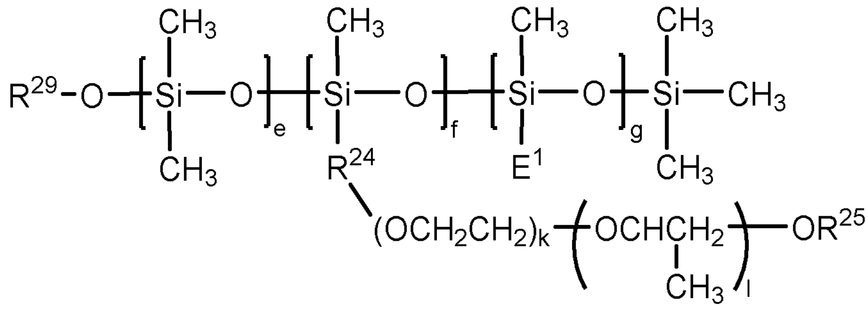

(2-1)

wherein $E^1$, $R^{24}$, $R^{25}$, e, f, and g are the same as above, $R^{29}$ represents a monovalent hydrocarbon group having 1 or more and 4 or less carbon atoms, or a trimethylsilyl group, k is a number of 1 or more and 50 or less, 1 is a number of 0 or more and 50 or less, and is preferably 1 or more and 50 or less.

<22> The cosmetic composition according to any one of <6>, <7>, and <19> to <21>, wherein the component (C2) is at least one selected from the group consisting of an aminopolyether-modified silicone having a structure represented by the following general formula (3-1) and an aminopolyether-modified silicone having a structure represented by the following general formula (3-2),

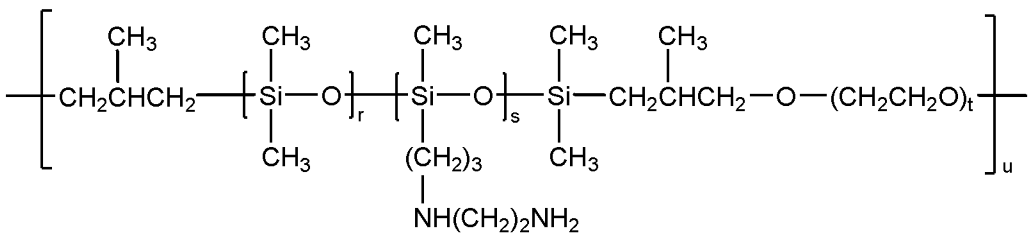

(3-1)

wherein r, s, t and u are the same as above,

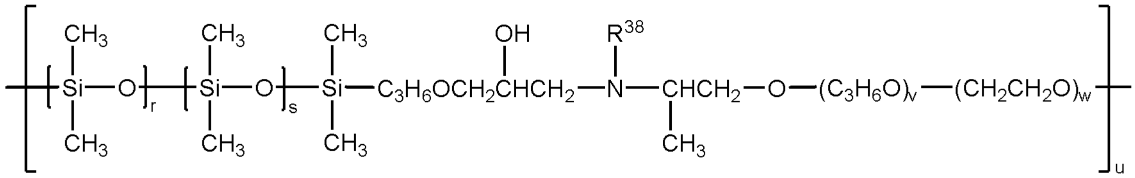

(3-2)

wherein $R^{38}$, r, s and u are the same as above, v is a number of 0 or more and 50 or less, preferably 2 or more and 50 or less, w is a number of 2 or more and 100 or less, v+w is a number of 2 or more and 100 or less, preferably 4 or more and 80 or less, more preferably 10 or more and 50 or less.

<23> The cosmetic composition according to <22>, wherein the component (C2) is at least one selected from the group consisting of an aminopolyether-modified silicone having a structure represented by the general formula (3-1) and an aminopolyether-modified silicone having a structure represented by the general formula (3-2), and is preferably an aminopolyether-modified silicone having a structure represented by the general formula (3-1).

<24> The cosmetic composition according to any one of <1>, and <8> to <23>, wherein a ratio of the content by mass of the component (C) to the total content by mass of the components (A) to (C) in the cosmetic composition, [(C)/((A)+(B)+(C))] is preferably 0.1 to 75%, more preferably 1 to 75%, even more preferably 2 to 75%, further more preferably 5 to 75%, further more preferably 5 to 50% further more preferably 7 to 35%, further more preferably 7 to 25%, further more preferably 9 to 25%.

<25> The cosmetic composition according to any one of <1>, and <8> to <24>, wherein a ratio of the content by mass of the component (A) to the total content by mass of the component (A) and the component (B) in the cosmetic composition, [(A)/((A)+(B))] is preferably 10 to 99%, more preferably 20 to 95%, even more preferably 20 to 90%, further more preferably 25 to 90%, further more preferably 30 to 90%, further more preferably 35 to 90%, further more preferably 35 to 88%.

<26> The cosmetic composition according to any one of <1>, and <8> to <25>, wherein the total content of the

components (A) to (C) in the cosmetic composition is preferably 0.1 to 50% by mass, more preferably 0.5 to 36% by mass, even more preferably 1 to 25% by mass, further more preferably 2 to 25% by mass, further more preferably 3 to 25% by mass, further more preferably 4 to 25% by mass, further more preferably 4 to 22% by mass, further more preferably 5 to 22% by mass.

<27> The cosmetic composition according to any one of <1> to <26>, wherein the content of the component (A) in the cosmetic composition is preferably 0.1 to 25% by mass, more preferably 0.1 to 20% by mass, even more preferably 0.5 to 20% by mass, further more preferably 1 to 20% by mass, further more preferably 2 to 15% by mass, further more preferably 2.5 to 15% by mass.

<28> The cosmetic composition according to any one of <1> to <27>, wherein the content of the component (B) in the cosmetic composition is preferably 0.1 to 25% by mass, more preferably 0.5 to 15% by mass, even more preferably 0.5 to 12% by mass, further more preferably 0.8 to 10% by mass, further more preferably 1 to 10% by mass, further more preferably 1 to 8% by mass.

<29> The cosmetic composition according to any one of <1> to <28>, wherein the content of the component (C) in the cosmetic composition is preferably 0.01 to 25% by mass, more preferably 0.1 to 15% by mass, even more preferably 0.5 to 12% by mass, further more preferably 0.5 to 10% by mass, further more preferably 0.6 to 10% by mass, further more preferably 0.7 to 10% by mass, further more preferably 0.7 to 5% by mass, further more preferably 0.5 to 5% by mass.

<30> The cosmetic composition according to any one of <1> to <29>, further containing a functional powder as a component (D).

<31> The cosmetic composition according to any one of <1> to <30>, further containing a solvent as a component (E).

<32> The cosmetic composition according to <31>, wherein the component (E) contains at least one selected from the group consisting of dimethylpolysiloxane having a viscosity at 25°C of 10 $mm^2/s$ or less, methyltrimethicone, methylphenylpolysiloxane having a viscosity at 25°C of 20 $mm^2/s$ or less, pentane, isopentane, hexane, isohexene, heptane, isoheptane, decane, isodecane, dodecane, isododecane, tetradecane, isotetradecane, tridecane, isotridecane and light liquid isoparaffin, preferably at least one selected from the group consisting of dimethylpolysiloxane having a viscosity at 25°C of 5 $mm^2/s$ or less, methyltrimethicone, isodecane, isododecane, isotetradecane and light liquid isoparaffin, more preferably at least one selected from the group consisting of isodecane, isododecane, isotetradecane and light liquid isoparaffin.

<33> The cosmetic composition according to <31> or <32>, wherein the component (E) preferably contains a volatile alcohol solvent and at least one of a volatile hydrocarbon solvent or a volatile silicone solvent, more preferably contains at least one selected from the group consisting of ethanol, 1-propanol, 2-propanol, 1-butanol and 2-butanol, and at least one selected from the group consisting of dimethylpolysiloxane having a viscosity at 25°C of 10 $mm^2/s$ or less, methyltrimethicone, methylphenylpolysiloxane having a viscosity at 25°C of 20 $mm^2/s$ or less, pentane, isopentane, hexane, isohexene, heptane, isoheptane, decane, isodecane, dodecane, isododecane, tetradecane, isotetradecane, tridecane, isotridecane, and light liquid isoparaffin, even more preferably contains ethanol and at least one selected from the group consisting of dimethylpolysiloxane having a viscosity at 25°C of 5 $mm^2/s$ or less, methyltrimethicone, isodecane, isododecane, isotetradecane and light liquid isoparaffin, further more preferably contains ethanol and at least one selected from the group consisting of isodecane, isododecane, isotetradecane and light liquid isoparaffin.

<34> The cosmetic composition according to any one of <31> to <33>, wherein the content of the component (E) in the cosmetic composition is preferably 1 to 99% by mass, more preferably 5 to 99% by mass, even more preferably 10 to 99% by mass, further more preferably 10 to 95% by mass, further more preferably 10 to 90% by mass.

<35> The cosmetic composition according to any one of <30> to <34>, wherein the content of the component (D) in the cosmetic composition is preferably 0.01 to 50% by mass, more preferably 0.1 to 50% by mass, even more preferably 0.2 to 30% by mass, further more preferably 0.3 to 30% by mass.

<36> The cosmetic composition according to any one of <1> to <35>, which is a hair cosmetic composition.

<37> The cosmetic composition according to any one of <1> to <36>, which is a hair dye composition.

<38> A method for treating a keratin substance, including a step of applying the cosmetic composition of any of <1> to <37> to a keratin substance and then drying it.

<39> A method for treating hair, including a step of applying the hair cosmetic composition of <36> to hair and then drying it.

<40> A method for dyeing hair, including a step of applying the hair dye composition of <37> to hair and then drying it.

<41> A cosmetic kit provided with at least two compositions, wherein:

the cosmetic composition obtained by mixing all the compositions constituting the cosmetic kit contains the following components (A) to (C),

(A) a silicone film-forming agent,

(B) a high-molecular-weight organopolysiloxane,

(C) an organopolysiloxane having a polyalkylene oxide moiety and a cationic group, other than the component (A) and the component (B).

<42> A cosmetic kit provided with at least two compositions, wherein:
the cosmetic composition obtained by mixing all the compositions constituting the cosmetic kit contains the following components (A) to (C),

(A) 0.1 to 25% by mass of a silicone resin represented by an average formula, $(R^1)_mSiO_{(4-m)/2}$

wherein $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3,
which contains a Q unit represented by $SiO_{4/2}$ and an M unit represented by $(R^1)_3SiO_{1/2}$,

(B) 0.1 to 25% by mass of an organopolysiloxane represented by the following general formula (1),

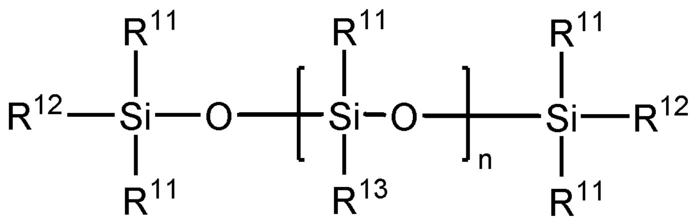 (1)

wherein $R^{11}$ each independently represents a hydrocarbon group having 1 or more and 6 or less carbon atoms, $R^{12}$ each independently represents a hydroxy group, an alkoxy group having 1 or more and 6 or less carbon atoms, or a hydrocarbon group having 1 or more and 6 or less carbon atoms, $R^{13}$ represents a hydrocarbon group having 1 or more and 6 or less carbon atoms, or a primary to tertiary amino group-containing group, n indicates a degree of polymerization, and is a number of 800 to 15,000, and n's $R^{13}$'s can be the same as or different from each other,
(C) 0.01 to 25% by mass of an organopolysiloxane having a polyalkylene oxide moiety and a cationic group, wherein the carbon number of the alkylene oxide in the polyalkylene oxide moiety is 1 to 6, the alkylene oxide average addition molar number is 2 to 100, and the cationic group is at least one selected from the group consisting of a primary amino group, a secondary amino group and a tertiary amino group.

<43> A cosmetic kit provided with at least two compositions, wherein:
the cosmetic composition obtained by mixing all the compositions constituting the cosmetic kit contains the following components (A) to (C),

(A) a silicone resin represented by $[SiO_{4/2}]_c[(R^1)_3SiO_{1/2}]_d$ wherein c and d each are an average repeating unit number and $c>0$ and $d>0$,
(B) an organopolysiloxane represented by the following general formula (1),

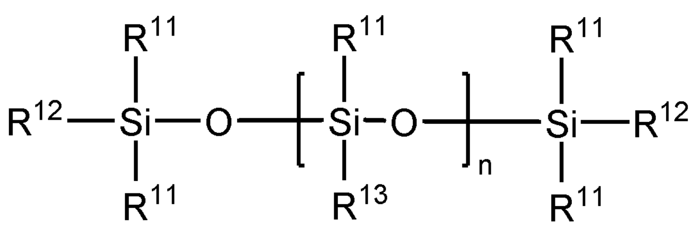 (1)

wherein $R^{11}$ each independently represents a hydrocarbon group having 1 or more and 6 or less carbon atoms, $R^{12}$ each independently represents a hydroxy group, an alkoxy group having 1 or more and 6 or less carbon atoms, or a hydrocarbon group having 1 or more and 6 or less carbon atoms, $R^{13}$ represents a hydrocarbon group having 1 or more and 6 or less carbon atoms, or a primary to tertiary amino group-containing group, n indicates a degree of polymerization, and is a number of 1,400 to 10,000, and n's $R^{13}$'s can be the same as or different from each other,
(C) an aminopolyether-modified silicone having a repeating unit represented by the following general formula (3),

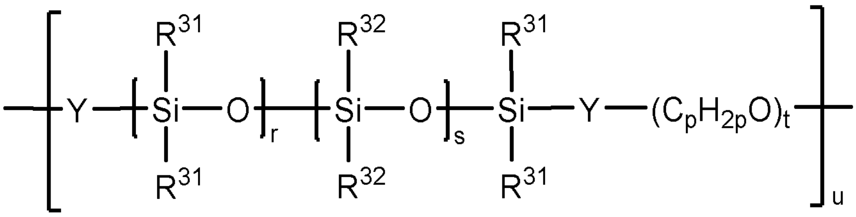 (3)

wherein $R^{31}$ represents a monovalent hydrocarbon group having 1 or more and 6 or less carbon atoms, $R^{32}$ represents any of $R^{31}$ or $E^2$, $E^2$ represents a monovalent group represented by $-R^{33}-Z^2$ (where $R^{33}$ represents a single bond, or a divalent hydrocarbon group having 1 or more and 20 or less carbon atoms, $Z^2$ represents a primary to tertiary amino group-containing group), Y represents a single bond, or a divalent group having 1 or more and 12 or less carbon atoms, in the case where all $R^{32}$'s are $R^{31}$'s, Y is an amino group-containing divalent group, in the case where all Y's are divalent groups not containing an amino group, at least one $R^{32}$ is $E^2$,

r is a number of 1 or more, s is a number of 1 or more, t is a number of 2 or more and 100 or less, u is a number of 1 or more. p is a number of 2 or more and 10 or less. The bonding order of the parenthesized structural units is not limited, and the bonding form thereof may be in a block form or a random form, t's $(C_pH_{2p}O)$s can be the same or different, plural $R^{31}$'s, $R^{32}$'s, $E^2$'s and Y's can be the same or different.

<44> The cosmetic kit according to <42> or <43>, wherein a ratio by mass of the content of the component (C) to the total content of the components (A) to (C) in the cosmetic composition obtained by mixing all the compositions in the cosmetic kit, [(C)/(A)+(B)+(C))] is 7% or more and 35% or less, a ratio by mass of the content of the component (A) to the total content of the component (A) and the component (B) in the cosmetic composition obtained by mixing all the compositions, [(A)/(A)+(B))] is 20% or more and 95% or less, and the total content by mass of the components (A) to (C) in the cosmetic composition obtained by mixing all the compositions is 1% by mass or more and 25% by mass or less.

<45> The cosmetic kit according to <42> or <43>, wherein a ratio by mass of the content of the component (C) to the total content of the components (A) to (C) in the cosmetic composition obtained by mixing all the compositions in the cosmetic kit, [(C)/(A)+(B)+(C))] is 9% or more and 25% or less, a ratio by mass of the content of the component (A) to the total content of the component (A) and the component (B) in the cosmetic composition obtained by mixing all the compositions, [(A)/(A)+(B))] is 20% or more and 95% or less, and the total content by mass of the components (A) to (C) in the cosmetic composition obtained by mixing all the compositions is 4% by mass or more and 22% by mass or less.

<46> A method for forming a film on the surface of a keratin substance, including the following step (I) to step (III) in that order,

Step (I): a step of temporarily compatibilizing or dispersing a composition containing the following components (A) to (C),

(A) a silicone film-forming agent,
(B) a high-molecular-weight organopolysiloxane,
(C) an organopolysiloxane having a polyalkylene oxide moiety and a cationic group, other than the component (A) and the component (B),

Step (II): a step of applying the composition prepared in the step (I) to the surface of a keratin substance,
Step (III): a step of drying the composition applied to the surface of a keratin substance in the step (II).

<47> The method according to <46>, wherein the film has a multilayered structure.

<48> A film to cover a surface of a keratin substance, wherein:
the film is a multilayer-structured film containing the following components (A) to (C), having a layer containing the component (B) in the outermost surface of the film, and having a layer containing the component (C) on the interface side to the keratin substance,

(A) a silicone film-forming agent,
(B) a high-molecular-weight organopolysiloxane,
(C) an organopolysiloxane having a polyalkylene oxide moiety and a cationic group, other than the component (A) and the component (B),

<49> The film according to <48>, further having a layer containing the component (A) between the layer containing the component (B) and the layer containing the component (C).
<50> The method according to <46> or <47>, or the film according to <48> or <49>, wherein the components (A) to (C) are the following,

(A) 0.1 to 25% by mass of a silicone resin represented by an average formula, $(R^1)_mSiO_{(4-m)/2}$

wherein $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3,
which contains a Q unit represented by $SiO_{4/2}$ and an M unit represented by $(R^1)_3SiO_{1/2}$,

(B) 0.1 to 25% by mass of an organopolysiloxane represented by the following general formula (1),

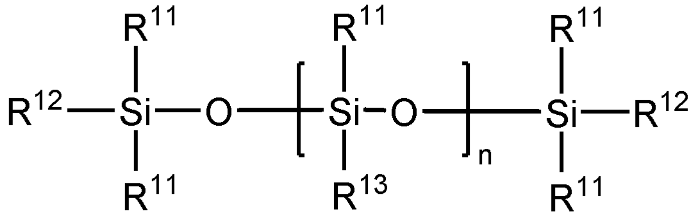 (1)

wherein $R^{11}$ each independently represents a hydrocarbon group having 1 or more and 6 or less carbon atoms, $R^{12}$ each independently represents a hydroxy group, an alkoxy group having 1 or more and 6 or less carbon atoms, or a hydrocarbon group having 1 or more and 6 or less carbon atoms, $R^{13}$ represents a hydrocarbon group having 1 or more and 6 or less carbon atoms, or a primary to tertiary amino group-containing group, n indicates a degree of polymerization, and is a number of 800 to 15,000, and n's $R^{13}$'s can be the same as or different from each other,
(C) 0.01 to 25% by mass of an organopolysiloxane having a polyalkylene oxide moiety and a cationic group, wherein the carbon number of the alkylene oxide in the polyalkylene oxide moiety is 1 to 6, the alkylene oxide average addition molar number is 2 to 100, and the cationic group is at least one selected from the group consisting of a primary amino group, a secondary amino group and a tertiary amino group.

<51>
The method or the film according to any one of <46> to <50>, wherein the components (A) to (C) are the following,

(A) a silicone resin represented by $[SiO_{4/2}]_c[(R^1)_3SiO_{1/2}]_d$ wherein c and d each are an average repeating unit number and c>0 and d>0,
(B) an organopolysiloxane represented by the following general formula (1),

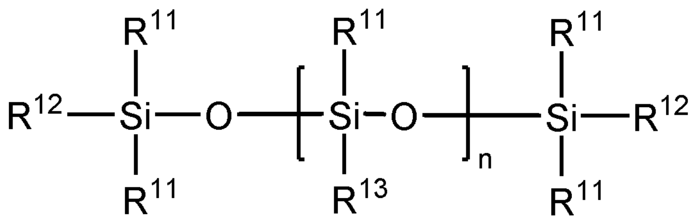 (1)

wherein $R^{11}$ each independently represents a hydrocarbon group having 1 or more and 6 or less carbon atoms, $R^{12}$ each independently represents a hydroxy group, an alkoxy group having 1 or more and 6 or less carbon atoms, or a hydrocarbon group having 1 or more and 6 or less carbon atoms, $R^{13}$ represents a hydrocarbon group having 1 or more and 6 or less carbon atoms, or a primary to tertiary amino group-containing group, n indicates a degree of polymerization, and is a number of 1,400 to 10,000, and n's $R^{13}$'s can be the same as or different from each other,
(C) an aminopolyether-modified silicone having a repeating unit represented by the following general formula (3),

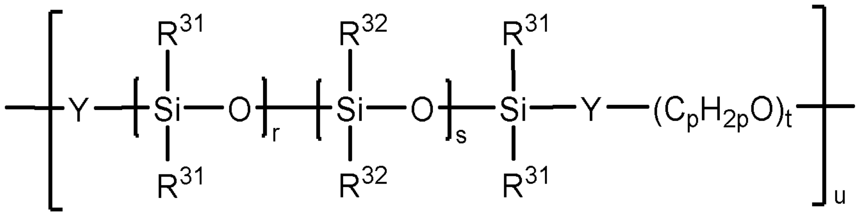 (3)

wherein $R^{31}$ represents a monovalent hydrocarbon group having 1 or more and 6 or less carbon atoms, $R^{32}$ represents any of $R^{31}$ or $E^2$, $E^2$ represents a monovalent group represented by $-R^{33}-Z^2$ (where $R^{33}$ represents a single bond, or a divalent hydrocarbon group having 1 or more and 20 or less carbon atoms, $Z^2$ represents a primary to tertiary amino group-containing group), Y represents a single bond, or a divalent group having 1 or more and 12 or less carbon atoms, in the case where all $R^{32}$'s are $R^{31}$'s, Y is an amino group-containing divalent group, in the case where all Y's are divalent groups not containing an amino group, at least one $R^{32}$ is $E^2$,

r is a number of 1 or more, s is a number of 1 or more, t is a number of 2 or more and 100 or less, u is a number of 1 or more, p is is a number of 2 or more and 10 or less, the bonding order of the parenthesized structural units is not limited, and the bonding form thereof may be in a block form or a random form, t's $(C_pH_{2p}O)$s can be the same or different, plural $R^{31}$'s, $R^{32}$'s, $E^2$'s and Y's can be the same or different.

<52> The method or the film according to any one of <46> to <51>, wherein a ratio by mass of the content of the component (C) to the total content of the components (A) to (C), [(C)/((A)+(B)+(C))] in the composition or the film is 7% or more and 35% or less, a ratio by mass of the component (A) to the total content of the component (A) and the component (B), [(A)/((A)+(B))] is 20% or more and 95% or less, and the total content of the components (A) to (C) is 1% by mass or more and 25% by mass or less.

<53> The method or the film according to any one of <46> to <52>, wherein a ratio by mass of the content of the component (C) to the total content of the components (A) to (C), [(C)/((A)+(B)+(C))] in the composition or the film is 9% or more and 25% or less, a ratio by mass of the component (A) to the total content of the component (A) and the component (B), [(A)/((A)+(B))] is 20% or more and 95% or less, and the total content of the components (A) to (C) is 4% by mass or more and 22% by mass or less.

Examples

Hereinunder the present invention is described with reference to Examples, but the present invention is not restricted to the range of Examples. In these Examples, various measurements and evaluations were carried out according to the following methods.

<Preparation of Hair Bundles for Evaluation>

Human gray hair (100%) bundles (by Beaulax Co., Ltd., length 10 cm, mass 1 g) were shampooed with the following model shampoo having a formulation mentioned below, then rinsed with warm water at 40°C, and fully dried to prepare hair bundles for evaluation.

(Model Shampoo Formulation)

| Ingredient | (mass%) |
|---|---|
| Polyoxyethylene(2) lauryl ether sodium sulfate (*1) | 15.5 |
| Lauric acid diethanolamide (*2) | 1.5 |
| Tetrasodium edetate | 0.3 |
| Sodium benzoate | 1.43 |
| Pure water | balance |
| Total | 100.0 |

*1: 57.4% by mass as Emal 227 (by Kao Corporation, active ingredient 27% by mass)
*2: Aminon L-02 (by Kao Corporation)

<Feel in dry state>

The hair cosmetic composition of each example was uniformly mixed, and 0.3 g thereof was applied to the hair bundle for evaluation, and then this was dried for 30 seconds with a drier ("P2-D250" by Hitachi Limited, setting HIGH) by applying hot air from a position separated by 18 cm from the hair bundle, and was thereafter further dried with hot air for 30 seconds while kept combed for hair treatment. After dried, expert panelists organoleptically evaluated the feel of the treated hair bundles according to the following criteria, and a total point of N = 3 was calculated. A total point of 6 or more means an acceptable feel, 9 or more means a good feel and 12 or more means a better feel.

5: Not sticky at all.
4: Sticky little.
3: Sticky slightly.
2: Sticky.
1: Extremely sticky.

<Feel during shampooing>

The hair cosmetic composition of each example was uniformly mixed, and 0.3 g thereof was applied to the hair bundle for evaluation, and then this was dried for 30 seconds with a drier ("P2-D250" by Hitachi Limited, setting HIGH) by applying hot air from a position separated by 18 cm from the hair bundle, and was thereafter further dried with hot air for 30 seconds while kept combed for hair treatment. After treated, the hair bundles were shampooed with the model shampoo having a formulation mentioned above, then rinsed with warm water at 40°C, and expert panelists organoleptically evaluated the feel of the resultant hair bundles according to the following criteria. A point 2 or more means a good feel, and 3 or more means a better feel.

3: The hair bundle readily became unraveled, and gave a light feel.
2: When combed with fingers, the hair bundle became unraveled and gave a slightly light feel.
1: The hair bundle was extremely hardly unraveled, and gave a heavy feel.

<Feel in dry state after 7-time shampooing>

The hair bundles treated in the same manner as in the above "feel in dry state" were shampooed with the model shampoo having a formulation as above, rinsed with warm water at 40°C and dried. The process was repeated 7 times. After shampooing 7 times, the feel of the dried hair bundles was organoleptically evaluated in the same manner as above.

<Shampooing Resistance (difficulty of discoloration in 7-time shampooing)

Regarding the hair cosmetic composition of Examples 1 to 20 and Comparative Example 1, in order that the formed film could be visually confirmed, 0.15 g of 15 mass% carbon black dispersion was added to 6 g of the hair cosmetic composition of each example, and uniformly mixed to prepare compositions for evaluation.
Regarding the hair cosmetic composition of Examples 21 to 25 containing a pigment as the component (D), the ingredients shown in the table were uniformly mixed to prepare hair cosmetic compositions for evaluation.
0.3 g of the composition was applied to the hair bundle for evaluation, and then this was dried for 30 seconds with a drier ("P2-D250" by Hitachi Limited, setting HIGH) by applying hot air from a position separated by 18 cm from the hair bundle, and was thereafter further dried with hot air for 30 seconds while kept combed for hair treatment.
The treated hair bundle was analyzed with a color difference meter (CR-400 by Konica Minolta, Inc.) in a CIE color system (L*,a*,b*). Then, this was shampooed with the model shampoo having a formulation mentioned above, rinsed with warm water at 40°C and dried. The process was repeated 7 times. After shampooing 7 times, the dried hair bundle was analyzed with the color difference meter in the same manner as above, and according to the following equation, a color difference (ΔE*) from the hair bundle before shampooing was calculated. L*, a* and b* were measured at different 6 points on the hair bundle (each at 2 central points of each region obtained by equally dividing the hair bundle into three in the length direction), and the found data were averaged to give an average value.

$$\Delta E^* = \sqrt{(L_1^* - L_0^*)^2 + (a_1^* - a_0^*)^2 + (b_1^* - b_0^*)^2}$$

wherein:

$L_0$*, $a_0$*, $b_0$*: found data of hair bundle after treatment (before shampooing),
$L_1$*, $a_1$*, $b_1$*: found data of hair bundle after 7-time shampooing
ΔE*<15 means that the treated hair is considered to have good shampooing resistance and to be excellent in color sustainability. ΔE*<10 means better, and ΔE*<5 means further better.

<Rubfastness (difficulty of color transfer)>

Regarding the hair cosmetic composition of Examples 1 to 20 and Comparative Example 1, in order that the formed film could be visually confirmed, 0.15 g of 15 mass% carbon black dispersion was added to 6 g of the hair cosmetic composition of each example, and uniformly mixed to prepare compositions for evaluation.
Regarding the hair cosmetic composition of Examples 21 to 25 containing a pigment as the component (D), the ingredients shown in the table were uniformly mixed to prepare hair cosmetic compositions for evaluation.
0.3 g of the composition was applied to the hair bundle for evaluation, and then this was dried for 30 seconds with a drier ("P2-D250" by Hitachi Limited, setting HIGH) by applying hot air from a position separated by 18 cm from the hair bundle, and was thereafter further dried with hot air for 30 seconds while kept combed for hair treatment.
Next, a qualitative filter disc No. 2 (150 mmΦ, by ADVANTEC Corporation) was folded half, and the treated hair bundle was sandwiched therein. While a weight of 600 g was put on this, the hair bundle was pulled out in about 1 second, and this operation was repeated for a total of 3 times.
After used, the filter disc was scanned, and using an image processing software "Image J", the area onto which carbon black or pigment had adhered was determined, and the adhesion ratio {(adhesion area) ÷ (filter area) × 100} (%) was calculated and shown in the table. A smaller value of adhesion ratio means that color transfer to the filter paper is smaller and the tested sample has better rubfastness. An adhesion ratio of 1.70 (%) or less means good, 0.95 (%) or less means better, and 0.60 (%) or less means further better.

<Rubfastness after 7-time shampooing>

The hair bundle treated in the same manner as in the above "rubfastness (difficulty of color transfer)" was shampooed with the model shampoo having a formation as above, rinsed with warm water at 40°C and dried. The process was repeated for a total of 7 times. The hair bundle shampooed and dried 7 times was evaluated for the rubfastness according to the same method as above.

Examples 1 to 20, Comparative Example 1 (preparation and evaluation of hair cosmetic composition)

Ingredients shown in Tables 1 to 6 were blended according to the formulation described in each Table, and then mixed until uniform to prepare hair cosmetic compositions. The resultant hair cosmetic compositions were evaluated according to the above-mentioned methods. The results are shown in Tables 1 to 6.
The blending amount (mass%) shown in Tables is an active ingredient amount.

Table 1

| (mass%) | | | Example 1 | Comparative Example 1 |
|---|---|---|---|---|
| (A) | (A1-2) Trimethylsiloxysilicate | X-21-5595 *1 | 6 | 8 |
| | | SR1000 *2 | | |
| | (A1-2) Trifluoropropyldimethyltrimethylsiloxysilicate | XS66-118226 *3 | | |
| | (A1-2) (Trimethylsiloxysilicate/dimethiconol) crosspolymer | FC-5002 *4 | | |
| | (A2-1) (Acrylates/polytrimethylsiloxymethacrylate) copolymer | FA-4002ID *5 | | |
| | (A1-1) Polypropylsilsesquioxane | 680 Fluid *6 | | |

(continued)

| (mass%) | | | Example 1 | Comparative Example 1 |
|---|---|---|---|---|
| (B) | High-molecular-weight dimethylpolysiloxane viscosity 100,000 $mm^2/s$ | KF-96H-100,000cs *7 | | |
| | High-molecular-weight dimethylpolysiloxane viscosity 1,000,000 $mm^2/s$ | KF-96H-1,000,000cs *8 | | |
| | High-molecular-weight dimethylpolysiloxane viscosity 20,000,000 $mm^2/s$ | Silsoft B3020 *9 | 3 | 4 |
| | High-molecular-weight dimethylpolysiloxane viscosity 30,000,000 $mm^2/s$ | X-25-9074 *10 | | |
| | High-molecular-weight dimethiconol | X-21-5666 *11 | | |
| | High-molecular-weight amino oil | KF-8018 *12 | | |
| (C) | (Bisisobutyl PEG-15/amodimethicone) copolymer | SS-3588 *13 | 3 | 0 |
| (E) | Isododecane | Marukasol R *14 | Balance | Balance |
| Total | | | 100 | 100 |
| Total content of (A)+(B)+(C) (mass%) | | | 12 | 12 |
| Ratio by mass of content of (C) in (A)+(B)+(C) (%) | | | 25.0 | 0.0 |
| Ratio by mass of content of (A) in (A)+(B) (%) | | | 66.7 | 66.7 |
| Evaluation Results | Rubfastness (difficulty of color transfer) | Adhesion ratio to filter (%) | 0.57 | 1.95 |
| | Feel in dry state | No stickiness | 15 | 3 |
| | Feel during shampooing | Easy to unravel | 3 | 1 |
| | Shampooing resistance (difficulty of discoloration by shampooing) | $\Delta E^*$ after 7-time shampooing | 0.5 | - |
| | Rubfastness after 7-time shampooing (difficulty of color transfer) | Adhesion ratio to filter (%) | 0.49 | - |
| | Feel in dry after 7-time shampooing | No stickiness | 15 | - |

Table 2

| (mass%) | | | Example 2 | Example 3 | Example 4 | Example 1 | Example 5 |
|---|---|---|---|---|---|---|---|
| (A) | (A1-2) Trimethylsiloxysilicate | X-21-5595 *1 | 7.84 | 7.6 | 7.0 | 6 | 4 |
| | | SR1000 *2 | | | | | |
| | (A1-2) Trifluoropropyldimethyltrimethylsiloxysilicate | XS66-B8226 *3 | | | | | |
| | (A1-2) (Trimethylsiloxysilicate/dimethiconol) crosspolymer | FC-5002 *4 | | | | | |
| | (A2-1) (Acrylates/polytrimethylsiloxymethacrylate) copolymer | FA-4002ID *5 | | | | | |
| | (A1-1) Polypropylsilsesquioxane | 680 Fluid *6 | | | | | |
| (B) | High-molecular-weight dimethylpolysiloxane viscosity 100,000 $mm^2/s$ | KF-96H-100,000cs *7 | | | | | |
| | High-molecular-weight dimethylpolysiloxane viscosity 1,000,000 $mm^2/s$ | KF-96H-1,000,000cs *8 | | | | | |
| | High-molecular-weight dimethylpolysiloxane viscosity 20,000,000 $mm^2/s$ | Silsoft B3020 *9 | 3.92 | 3.8 | 3.5 | 3 | 2 |
| | High-molecular-weight dimethylpolysiloxane viscosity 30,000,000 $mm^2/s$ | X-25-9074 *10 | | | | | |
| | High-molecular-weight dimethiconol | X-21-5666 *11 | | | | | |
| | High-molecular-weight amino oil | KF-8018 *12 | | | | | |
| (C) | (Bisisobutyl PEG-15/amodimethicone) copolymer | SS-3588 *13 | 0.24 | 0.6 | 1.5 | 3 | 6 |
| (E) | Isododecane | Marukasol R *14 | Balance | Balance | Balance | Balance | Balance |
| Total | | | 100 | 100 | 100 | 100 | 100 |
| Total content of (A)+(B)+(C) (mass%) | | | 12 | 12 | 12 | 12 | 12 |
| Ratio by mass of content of (C) in (A)+(B)+(C) (%) | | | 2.0 | 5.0 | 12.5 | 25.0 | 50.0 |
| Ratio by mass of content of (A) in (A)+(B) (%) | | | 66.7 | 66.7 | 66.7 | 66.7 | 66.7 |

(continued)

| (mass%) | | | Example 2 | Example 3 | Example 4 | Example 1 | Example 5 |
|---|---|---|---|---|---|---|---|
| Evaluation Results | Rubfastness (difficulty of color transfer) | Adhesion ratio to filter (%) | 0.76 | 0.76 | 0.70 | 0.57 | 0.98 |
| | Feel in dry state | No stickiness | 6 | 8 | 13 | 15 | 15 |
| | Feel during shampooing | Easy to unravel | 2 | 2 | 3 | 3 | 3 |
| | Shampooing resistance (difficulty of discoloration by shampooing) | $\Delta E^*$ after 7-time shampooing | 2.1 | 1.8 | 0.8 | 0.5 | 2.0 |
| | Rubfastness after 7-time shampooing (difficulty of color transfer) | Adhesion ratio to filter (%) | 0.54 | 0.42 | 0.6 | 0.49 | 0.52 |
| | Feel in dry after 7-time shampooing | No stickiness | 6 | 8 | 15 | 15 | 15 |

Table 3

| (mass%) | | | Example 6 | Example 1 | Example 7 | Example 8 |
|---|---|---|---|---|---|---|
| (A) | (A1-2) Trimethylsiloxysilicate | X-21-5595 *1 | | 6 | | |
| | | SR1000 *2 | 1 | | 12 | 18 |
| | (A1-2) Trifluoropropyldimethyltrimethylsiloxysilicate | XS66-B8226 *3 | | | | |
| | (A1-2) (Trimethylsiloxysilicate/dimethiconol) crosspolymer | FC-5002 *4 | | | | |
| | (A2-1) (Acrylates/polytrimethylsiloxymethacrylate) copolymer | FA-4002ID *5 | | | | |
| | (A1-1) Polypropylsilsesquioxane | 680 Fluid *6 | | | | |
| (B) | High-molecular-weight dimethylpolysiloxane viscosity 100,000 $mm^2/s$ | KF-96H-100,000cs *7 | | | | |
| | High-molecular-weight dimethylpolysiloxane viscosity 1,000,000 $mm^2/s$ | KF-96H-1,000,000cs *8 | | | | |
| | High-molecular-weight dimethylpolysiloxane viscosity 20,000,000 $mm^2/s$ | Silsoft B3020 *9 | 0.5 | 3 | 6 | 9 |
| | High-molecular-weight dimethylpolysiloxane viscosity 30,000,000 $mm^2/s$ | X-25-9074 *10 | | | | |
| | High-molecular-weight dimethiconol | X-21-5666 *11 | | | | |
| | High-molecular-weight amino oil | KF-8018 *12 | | | | |
| (C) | (Bisisobutyl PEG-15/amodimethicone) copolymer | SS-3588 *13 | 0.5 | 3 | 6 | 9 |
| (E) | Isododecane | Marukasol R *14 | Balance | Balance | Balance | Balance |
| Total | | | 100 | 100 | 100 | 100 |
| Total content of (A)+(B)+(C) (mass%) | | | 2 | 12 | 24 | 36 |
| Ratio by mass of content of (C) in (A)+(B)+(C) (%) | | | 25.0 | 25.0 | 25.0 | 25.0 |
| Ratio by mass of content of (A) in (A)+(B) (%) | | | 66.7 | 66.7 | 66.7 | 66.7 |

(continued)

| (mass%) | | | Example 6 | Example 1 | Example 7 | Example 8 |
|---|---|---|---|---|---|---|
| Evaluation Results | Rubfastness (difficulty of color transfer) | Adhesion ratio to filter (%) | 1.30 | 0.57 | 0.49 | 0.90 |
| | Feel in dry state | No stickiness | 14 | 15 | 9 | 7 |
| | Feel during shampooing | Easy to unravel | 3 | 3 | 2 | 2 |
| | Shampooing resistance (difficulty of discoloration by shampooing) | ΔE* after 7-time shampooing | 6.7 | 0.5 | 0.5 | 0.6 |
| | Rubfastness after 7-time shampooing (difficulty of color transfer) | Adhesion ratio to filter (%) | 0.54 | 0.49 | 0.65 | 0.74 |
| | Feel in dry after 7-time shampooing | No stickiness | 15 | 15 | 9 | 7 |

Table 4

| (mass%) | | | Example 1 | Example 9 | Example 10 | Example 11 | Example 12 |
|---|---|---|---|---|---|---|---|
| (A) | (A1-2) Trimethylsiloxysilicate | X-21-5595 *1 | 6 | | | | |
| | | SR1000 *2 | | | | | |
| | (A1-2) Trifluoropropyldimethyltrimethylsiloxysilicate | XS66-B8226 *3 | | 6 | | | |
| | (A1-2) (Trimethylsiloxysilicate/dimethiconol) crosspolymer | FC-5002 *4 | | | 6 | | |
| | (A2-1) (Acrylates/polytrimethylsiloxymethacrylate) copolymer | FA-4002ID *5 | | | | 6 | |
| | (A1-1) Polypropylsilsesquioxane | 680 680 Fluid *6 | | | | | 7 |
| (B) | High-molecular-weight dimethylpolysiloxane viscosity 100,000 $mm^2/s$ | KF-96H-100,000cs *7 | | | | | |
| | High-molecular-weight dimethylpolysiloxane viscosity 1,000,000 $mm^2/s$ | KF-96H-1,000,000cs *8 | | | | | |
| | High-molecular-weight dimethylpolysiloxane viscosity 20,000,000 $mm^2/s$ | Silsoft B3020 *9 | 3 | 3 | 3 | 3 | 3.5 |
| | High-molecular-weight dimethylpolysiloxane viscosity 30,000,000 $mm^2/s$ | X-25-9074 *10 | | | | | |
| | High-molecular-weight dimethiconol | X-21-5666 *11 | | | | | |
| | High-molecular-weight amino oil | KF-8018 *12 | | | | | |
| (C) | (Bisisobutyl PEG-15/amodimethicone) copolymer | SS-3588 *13 | 3 | 3 | 3 | 3 | 1.5 |
| (E) | Isododecane | Marukasol R *14 | Balance | Balance | Balance | Balance | Balance |
| Total | | | 100 | 100 | 100 | 100 | 100 |
| Total content of (A)+(B)+(C) (mass%) | | | 12 | 12 | 12 | 12 | 12 |
| Ratio by mass of content of (C) in (A)+(B)+(C) (%) | | | 25.0 | 25.0 | 25.0 | 25.0 | 12.5 |
| Ratio by mass of content of (A) in (A)+(B) (%) | | | 66.7 | 66.7 | 66.7 | 66.7 | 66.7 |

(continued)

| (mass%) | | | Example 1 | Example 9 | Example 10 | Example 11 | Example 12 |
|---|---|---|---|---|---|---|---|
| Evaluation Results | Rubfastness (difficulty of color transfer) | Adhe sion ratio to filter (%) | 0.57 | 0.59 | 0.34 | 1.55 | 1.29 |
| | Feel in dry state | No stickiness | 15 | 14 | 14 | 15 | 15 |
| | Feel during shampooing | Easy to unravel | 3 | 3 | 3 | 3 | 3 |
| | Shampooing resistance (difficulty of discoloration by shampooing) | $\Delta E^*$ sham after 7-time pooing | 0.5 | 0.3 | 0.5 | 14.0 | 11.6 |
| | Rubfastness after 7-time shampooing (difficulty of color transfer) | Adhe sion ratio to filter (%) | 0.49 | 0.83 | 1.06 | 1.51 | 1.34 |
| | Feel in dry after 7-time shampooing | No stickiness | 15 | 14 | 14 | 15 | 15 |

Table 5

| (mass%) | | | Example 13 | Example 14 | Example 1 | Example 15 | Example 16 | Example 17 |
|---|---|---|---|---|---|---|---|---|
| (A) | (A1-2) Trimethylsiloxysilicate | X-21-5595 *1 | 6 | 6 | 6 | 6 | 6 | 6 |
| | | SR1000 *2 | | | | | | |
| | (A1-2) Trifluoropropyldimethyltrimethylsiloxysilicate | XS66-B8226 *3 | | | | | | |
| | (A1-2) (Trimethylsiloxysilicate/dimethiconol) crosspolymer | FC-5002 *4 | | | | | | |
| | (A2-1) (Acrylates/polytrimethylsiloxymethacrylate) copolymer | FA-4002ID *5 | | | | | | |
| | (A1-1) Polypropylsilsesquioxane | 680 Fluid *6 | | | | | | |
| (B) | High-molecular-weight dimethylpolysiloxane viscosity 100,000 $mm^2/s$ | KF-96H-100,000cs *7 | 3 | | | | | |
| | High-molecular-weight dimethylpolysiloxane viscosity 1,000,000 $mm^2/s$ | KF-96H-1,000,000cs *8 | | 3 | | | | |
| | High-molecular-weight dimethylpolysiloxane viscosity 20,000,000 $mm^2/s$ | SilsoftB3020 *9 | | | 3 | | | |
| | High-molecular-weight dimethylpolysiloxane viscosity 30,000,000 $mm^2/s$ | X-25-9074 *10 | | | | 3 | | |
| | High-molecular-weight dimethiconol | X-21-5666 *11 | | | | | 3 | |
| | High-molecular-weight amino oil | KF-8018 *12 | | | | | | 3 |
| (C) | (Bisisobutyl PEG-15/amodimethicone) copol ymer | SS-3588 *13 | 3 | 3 | 3 | 3 | 3 | 3 |
| (E) | Isododecane | Marukasol R *14 | Balance | Balance | Balance | Balance | Balance | Balance |
| Total | | | 100 | 100 | 100 | 100 | 100 | 100 |
| Total content of (A)+(B)+(C) (mass%) | | | 12 | 12 | 12 | 12 | 12 | 12 |
| Ratio by mass of content of (C) in (A)+(B)+(C) (%) | | | 25.0 | 25.0 | 25.0 | 25.0 | 25.0 | 25.0 |
| Ratio by mass of content of (A) in (A)+(B) (%) | | | 66.7 | 66.7 | 66.7 | 66.7 | 66.7 | 66.7 |

(continued)

| (mass%) | | | Example 13 | Example 14 | Example 1 | Example 15 | Example 16 | Example 17 |
|---|---|---|---|---|---|---|---|---|
| Evaluation Results | Rubfastness (difficulty of color transfer) | Adhesion ratio to filter (%) | 0.64 | 0.67 | 0.57 | 0.48 | 0.37 | 0.57 |
| | Feel in dry state | No stickiness | 13 | 13 | 15 | 14 | 13 | 14 |
| | Feel during shampooing | Easy to unravel | 3 | 3 | 3 | 3 | 3 | 3 |
| | Shampooing resistance (difficulty of discoloration by shampooing) | ΔE* after 7-time shampooing | 1.1 | 0.6 | 0.5 | 1.1 | 0.5 | 0.7 |
| | Rubfastness after 7-time shampooing (difficulty of color transfer) | Adhesion ratio to filter (%) | 0.48 | 0.64 | 0.49 | 0.36 | 0.33 | 0.48 |
| | Feel in dry after 7-time shampooing | No stickiness | 14 | 13 | 15 | 13 | 13 | 13 |

Table 6

| (mass%) | | | Example 18 | Example 19 | Example 4 | Example 20 |
|---|---|---|---|---|---|---|
| (A) | (A1-2) Trimethylsiloxysilicate | X-21-5595 *1 | 1.2 | 2.62 | 7.0 | 9.6 |
| | | SR1000 *2 | | | | |
| | (A1-2) Trifluoropropyldimethyltrimethylsiloxysilicate | XS66-B8226 *3 | | | | |
| | (A1-2) (Trimethylsiloxysilicate/dimethiconol) crosspolymer | FC-5002 *4 | | | | |
| | (A2-1) (Acrylates/polytrimethylsiloxymethacrylate) copolymer | FA-4002ID *5 | | | | |
| | (A1-1) Polypropylsilsesquioxane | 680 Fluid *6 | | | | |
| (B) | High-molecular-weight dimethylpolysiloxane viscosity 100,000 $mm^2/s$ | KF-96H-100,000cs *7 | | | | |
| | High-molecular-weight dimethylpolysiloxane viscosity 1,000,000 $mm^2/s$ | KF-96H-1,000,000cs *8 | | | | |
| | High-molecular-weight dimethylpolysiloxane viscosity 20,000,000 $mm^2/s$ | Silsoft B3020 *9 | 9.3 | 7.88 | 3.5 | 0.9 |
| | High-molecular-weight dimethylpolysiloxane viscosity 30,000,000 $mm^2/s$ | X-25-9074 *10 | | | | |
| | High-molecular-weight dimethiconol | X-21-5666 *11 | | | | |
| | High-molecular-weight amino oil | KF-8018 *12 | | | | |
| (C) | (Bisisobutyl PEG-15/amodimethicone) copolymer | SS-3588 *13 | 1.5 | 1.5 | 1.5 | 1.5 |
| (E) | Isododecane | Marukasol R *14 | Balance | Balance | Balance | Balance |
| Total | | | 100 | 100 | 100 | 100 |
| Total content of (A)+(B)+(C) (mass%) | | | 12 | 12 | 12 | 12 |
| Ratio by mass of content of (C) in (A)+(B)+(C) (%) | | | 12.5 | 12.5 | 12.5 | 12.5 |
| Ratio by mass of content of (A) in (A)+(B) (%) | | | 11.4 | 25.0 | 66.7 | 91.4 |

(continued)

| (mass%) | | | Example 18 | Example 19 | Example 4 | Example 20 |
|---|---|---|---|---|---|---|
| Evaluation Results | Rubfastness (difficulty of color transfer) | Adhesion ratio to filter (%) | 1.41 | 0.93 | 0.70 | 1.46 |
| | Feel in dry state | No stickiness | 12 | 11 | 13 | 15 |
| | Feel during shampooing | Easy to unravel | 3 | 3 | 3 | 3 |
| | Shampooing resistance (difficulty of discoloration by shampooing) | $\Delta E^*$ after 7-time shampooing | 3.0 | 0.4 | 0.8 | 2.0 |
| | Rubfastness after 7-time shampooing (difficulty of color transfer) | Adhesion ratio to filter (%) | 0.11 | 0.63 | 0.60 | 1.64 |
| | Feel in dry after 7-time shampooing | No stickiness | 13 | 13 | 15 | 15 |

Ingredients shown in the tables are as described below.

*1: X-21-5595, by Shin-Etsu Chemical Industry Co., Ltd., isododecane solution of trimethylsiloxysilicate (60 mass%)
*2: SR1000, by Momentive Performance Materials Corporation, trimethylsiloxysilicate
*3: XS66-B8226, by Momentive Performance Materials Corporation, trifluoropropyldimethyl/trimethylsiloxysilicate (50 mass%) cyclopentasiloxane solution
*4: DOWSIL FC-5002 IDD Resin Gum, by Dow Toray Corporation, (trimethylsiloxysilicate/dimethiconol) crosspolymer (40 mass%) isododecane solution
*5: DOWSIL FA 4002 ID Silicone Acrylate, by Dow Toray Corporation, (acrylates/polytrimethylsiloxymethacrylate) copolymer (30 mass%) isododecane solution
*6: DOWSIL 680 ID Fluid, by Dow Toray Corporation, polypropylsilsesquioxane (75 mass%) isododecane solution
*7: KF-96H-100,000cs, by Shin-Etsu Chemical Industry Co., Ltd., high-molecular-weight dimethylpolysiloxane, viscosity at 25°C: 100,000 $mm^2/s$
*8: KF-96H-1,000,000cs, by Shin-Etsu Chemical Industry Co., Ltd., high-molecular-weight dimethylpolysiloxane, viscosity at 25°C: 1,000,000 $mm^2/s$
*9: Silsoft B3020, by Momentive Performance Materials Corporation, high-molecular-weight dimethylpolysiloxane, viscosity at 25°C; 20,000,000 $mm^2/s$
*10: X-25-9074, by Shin-Etsu Chemical Industry Co., Ltd., high-molecular-weight dimethylpolysiloxane, viscosity at 25°C: 30,000,000 $mm^2/s$
*11: X-21-5666, by Shin-Etsu Chemical Industry Co., Ltd., high-molecular-weight dimethiconol (30 mass%) cyclopentanesiloxane solution, viscosity at 25°C: 3,500,000 $mm^2/s$
*12: X-8018, by Shin-Etsu Chemical Industry Co., Ltd., aminopropyl group-added high-molecular-weight dimethicone (10 mass%) cyclopentanesiloxane solution, degree of polymerization 3,000 to 4,000
*13: DOWSIL SS-3588 Fluid, by Dow Toray Corporation, (bisisobutyl PEG-15/amodimethicone) copolymer
*14: Marukasol R, by Maruzen Petrochemical Co., Ltd., isododecane

The degree of polymerization (P) of the components *7 to *11 can be calculated as follows. From the viscosity (η), the molecular weight (M) is derived according to the above-mentioned formula (4). Since the molecular weight of the base unit of dimethylpolysiloxane is 74, the degree of polymerization (P) is calculated according to the following formula (5).

$$P=M/74 \qquad (5)$$

As in Tables 1 to 6, when the cosmetic composition of the present invention was applied to keratin substances, a good feel was given in any of a dry state and a wet state, the composition was excellent in durability to friction, and even after washing 7 times, these effects were sustained.

<Confirmation of film forming condition>

Fig. 1 shows an elastic modulus analyzing image (analytical region 5 μm × 5 pm) of a hair cross section after treatment with the hair cosmetic composition of Example 1 that had been analyzed according to the following analysis method, and an enlarged image of the film part thereof. The elastic modulus analyzing image shows that the elastic modulus is low in the dark area and is high in the light region.

The film formed of the hair cosmetic composition of Example 1 is composed of trimethylsiloxysilicate of the component (A), high-molecular-weight organopolysiloxane of the component (B) and PEG/amodimethicone copolymer of the component (C).

As in Fig. 1, a region having a high elastic modulus appears in the intermediate part of the film of the elastic modulus analyzing image, which confirms that a mixed system of the components (A) to (C) underwent phase separation to form a multilayered structure.

The elastic modulus of the film in dry of the component (A) is higher than that of the components (B) and (C), from which it is presumed that the component (A) is eccentrically located in the intermediate part of the film.

On the other hand, the hair cosmetic composition of Example 1 gives a good feel in any of a dry state and a wet state, from which it is presumed that the component (B) having surface smoothness is eccentrically located in the outermost surface of the film. In addition, from durability to friction and durability to washing, it is presumed that the component (C) having high adhesiveness to a keratin substance is eccentrically located on the keratin substance side.

Further, since the elastic modulus in the film intermediate part of the elastic modulus analyzing image gently varies toward the film thickness direction, it is presumed that the layers containing the respective components are not

clearly separated from each other and that in the interlayer therebetween high-molecular chains of different components mutually invade thereinto to form an interface phase having a thickness where the concentration continuously varies.

As in the above, it is confirmed that, after applied to the surface of a keratin substance, the hair cosmetic composition of Example 1 undergoes phase separations of the components (A) to (C) to form a multilayered structure on the surface of the keratin substance.

[Preparation of Test Sample]

The hair cosmetic composition of Example 1 was uniformly mixed, and 0.3 g thereof was applied to the hair bundle for evaluation, and then dried with a drier for hair treatment. The treated hair bundle was immersed in a liquid photocurable resin composition (Aronix LCR D-800, by Toagosei Co., Ltd.) and irradiated with UV rays for 1 minute (LED fiber light source (LUXSPOT II), by JEOL Corporation), and thus every hair was buried in the resin one by one. The resin in which the treated hair had been buried was fixed on a 12 mmΦ-AFM sample stand (AFM specimen disc, by TED PELLA Corporation) using an adhesive. Using a single-edged trimming razor (T568, by GEM Corporation), the test sample face was exposed out, and using an ultramicrotome (EM UC7i, by Hitachi High Tech Corporation), the test sample face was scraped out.

[Test Method]

For the measurement, an atomic force microscope (MFP-3D, by Oxford Instrument Corporation) was used in an AMFM mode. As the cantilever, AC-160TS (spring constant 29.4 N/m, by Olympus Corporation) was used. The measurement environment was temperature of 25.5°C and humidity of 34 to 39%.

Examples 21 to 24 (preparation and evaluation of hair dye composition)

Ingredients shown in Table 7 were blended according to the formulation described in the Table, and then mixed until uniform to prepare hair dye compositions. The resultant hair dye compositions were evaluated according to the above-mentioned methods. The results are shown in Table 7.

The blending amount (mass%) shown in the Table is an active ingredient amount.

Table 7

| (mass%) | | | Example 21 | Example 22 | Example 23 | Example 24 |
|---|---|---|---|---|---|---|
| (A) | (A1-2) Trimethylsiloxysilicate | X-21-5595 *1 | 6 | 6 | 5.85 | 6.83 |
| | | SR1000 *2 | | | | |
| | (A1-2) Trifluoropropyldimethyltrimethylsiloxysilicate | XS66-B8226 *3 | | | | |
| | (A1-2) (Trimethylsiloxysilicate/dimethiconol) crosspolymer | FC-5002 *4 | | | | |
| | (A2-1) (Acrylates/polytrimethylsiloxymethacrylate) copolymer | FA-4002ID *5 | | | | |
| | (A1-1) Polypropylsilsesquioxane | 680 Fluid *6 | | | | |
| (B) | High-molecular-weight dimethylpolysiloxane viscosity 100,000 $mm^2/s$ | KF-96H-100,000cs *7 | | | | |
| | High-molecular-weight dimethylpolysiloxane viscosity 1,000,000 $mm^2/s$ | KF-96H-1,000,000cs *8 | | | | |
| | High-molecular-weight dimethylpolysiloxane viscosity 20,000,000 $mm^2/s$ | Silsoft B3020 *9 | 3 | 3 | 2.93 | 3.41 |
| | High-molecular-weight dimethylpolysiloxane viscosity 30,000,000 $mm^2/s$ | X-25-9074 *10 | | | | |
| | High-molecular-weight dimethiconol | X-21-5666 *11 | | | | |
| | High-molecular-weight amino oil | KF-8018 *12 | | | | |
| (C) | PEG/amodimethicone copolymer | SS-3588 *13 | 3 | 3 | 2.93 | 1.46 |
| (D) | Red No. 202 | BC Red No. 202 K *15 | 2 | 1 | | |
| | Pearl pigment | COLORONA BORDEAUX *16 | | 1 | | |
| | Carbon black | DK BLACK No. 2 *17 | | | 0.36 | 0.36 |
| (E) | Isododecane | Marukasol R *14 | Balance | Balance | Balance | Balance |
| Total | | | 100 | 100 | 100 | 100 |
| Total content of (A)+(B)+(C) (mass%) | | | 12 | 12 | 11.71 | 11.7 |
| Ratio by mass of content of (C) in (A)+(B)+(C) (%) | | | 25.0 | 25.0 | 25.0 | 12.5 |
| Ratio by mass of content of (A) in (A)+(B) (%) | | | 66.7 | 66.7 | 66.6 | 66.7 |

(continued)

| (mass%) | | | Example 21 | Example 22 | Example 23 | Example 24 |
|---|---|---|---|---|---|---|
| Evaluation Results | Rubfastness (difficulty of color transfer) | Adhesion ratio to filter (%) | 0.64 | 0.33 | 0.57 | 0.7 |
| | Feel in dry state | No stickiness | 15 | 15 | 15 | 13 |
| | Feel during shampooing | Easy to unravel | 3 | 3 | 3 | 3 |
| | Shampooing resistance (difficulty of discoloration by shampooing) | ΔE* after 7-time shampooing | 1.5 | 2.0 | 0.5 | 0.8 |
| | Rubfastness after 7-time shampooing (difficulty of color transfer) | Adhesion ratio to filter (%) | 0.74 | 0.48 | 0.49 | 0.6 |
| | Feel in dry after 7-time shampooing | No stickiness | 15 | 15 | 14 | 14 |

*15: BC Red No. 202 K, by Kishi Kasei Co., Ltd.
*16: COLORONA BORDEAUX, by EMD Chemicals Inc.
*17: DK BLACK No. 2, by Daito Kasei Kogyo Co., Ltd.

As in Table 7, when the hair dye composition of the present invention was applied to hair, a good feel was given in any of a dry state and a wet state, the composition was excellent in durability to friction, and even after washing 7 times, these effects were sustained.

Example 25 (preparation and evaluation of hair dye composition)

Ingredients shown in Table 8 were blended according to the formulation described in the Table, and then mixed until uniform to prepare a hair dye composition. The resultant hair dye composition was evaluated for rubfastness, feel in a dry state, feel during shampooing and shampooing resistance according to the above-mentioned methods.
Further, for confirming the sustainability of the effects of the composition on hair, the following shampooing resistance (difficulty of discoloration in 28-time shampooing), rubfastness after 28-time shampooing, and feel in a dry state after 28-time shampooing were evaluated. The results are shown in Table 8.
The blending amount (mass%) shown in the Table is an active ingredient amount.

<Shampooing Resistance (difficulty of discoloration in 28-time shampooing)>

The hair bundle treated in the same manner as in the above "shampooing resistance (difficulty of discoloration in 7-time shampooing)" was analyzed with the above-mentioned color difference meter in a CIE color system (L*,a*,b*). Then, this was shampooed with the model shampoo having a formulation mentioned above, rinsed with warm water at 40°C and dried. The process was repeated for a total of 28 times. After shampooing 28 times, the dried hair bundle was analyzed with the color difference meter in the same manner as above, and a color difference (ΔE*) from the hair bundle before shampooing was calculated in the same manner as above.

<Rubfastness after 28-time shampooing>

The hair bundle treated in the same manner as in the above "rubfastness (difficulty of color transfer)" was shampooed with the model shampoo having a formation as above, rinsed with warm water at 40°C and dried. The process was repeated for a total of 28 times. The hair bundle shampooed and dried 28 times was evaluated for the rubfastness according to the same method as above.

<Feel in dry state after 28-time shampooing>

The hair bundle treated in the same manner as in the above "feel in dry state" was shampooed with the model shampoo having a formulation as above, rinsed with warm water at 40°C and dried. The process was repeated 28 times. After shampooing 28 times, the feel of the dried hair bundle was evaluated in the same manner as above.

Table 8

| | (mass%) | | Example 25 |
|---|---|---|---|
| (A) | (A1-2) Trimethylsiloxysilicate | X-21-5595 *1 | 4 |
| | | SR1000 *2 | |
| | (A1-2) Trifluoropropyldimethyltrimethylsiloxysilicate | XS66-B8226 *3 | |
| | (A1-2) (Trimethylsiloxysilicate/dimethiconol) crosspolymer | FC-5002 *4 | |
| | (A2-1) (Acrylates/polytrimethylsiloxymethacrylate) copolymer | FA-4002ID *5 | |
| | (A1-1) Polypropylsilsesquioxane | 680 Fluid *6 | |

(continued)

| | (mass%) | | | Example 25 |
|---|---|---|---|---|
| (B) | High-molecular-weight dimethylpolysiloxane viscosity 100,000 $mm^2/s$ | | KF-96H-100,000cs *7 | |
| | High-molecular-weight dimethylpolysiloxane viscosity 1,000,000 $mm^2/s$ | | KF-96H-1,000,000cs *8 | |
| | High-molecular-weight dimethylpolysiloxane viscosity 20,000,000 $mm^2/s$ | | SilsoftB3020 *9 | |
| | High-molecular-weight dimethylpolysiloxane viscosity 30,000,000 $mm^2/s$ | | X-25-9074 *10 | 2 |
| | High-molecular-weight dimethiconol | | X-21-5666 *11 | |
| | High-molecular-weight amino oil | | KF-8018 *12 | |
| (C) | PEG/amodimethicone copolymer | | SS-3588 *13 | 2 |
| (D) | Carbon black | | DK BLACK No. 2 *17 | 0.75 |
| (E) | Isododecane | | Marukasol R * 14 | Balance |
| Total | | | | 100 |
| Total content of (A)+(B)+(C) (mass%) | | | | 8 |
| Ratio by mass of content of (C) in (A)+(B)+(C) (%) | | | | 25.0 |
| Ratio by mass of content of (A) in (A)+(B) (%) | | | | 66.7 |
| Evaluation Results | Rubfastness (difficulty of color transfer) | Adh esion ratio to filter (%) | | 0.49 |
| | Feel in dry state | No stickiness | | 15 |
| | Feel during shampooing | Easy to unravel | | 3 |
| | Shampooing resistance (difficulty of discoloration by shampooing) | ΔE* after 7-time shampooing | | 0.7 |
| | | ΔE* after 28-time shampooing | | 1.6 |
| | Rubfastness after 28-time shampooing (difficulty of color transfer) | Adhesion ratio to filter (%) | | 0.47 |
| | Feel in dry after 28-time shampooing | No stickiness | | 15 |

As in Table 8, when the hair dye composition of the present invention was applied to hair, a good feel was given in any of a dry state and a wet state, the composition was excellent in durability to friction, and even after washing 28 times, these effects were sustained. From this, it is considered that, in shampooing once a day, the hair dye effect can be sustained for about one month.

Industrial Applicability

According to the present invention, there can be provided a cosmetic composition, which, when applied to keratin substances such as skin or hair, can give a good feel in any of a dry state and a wet state, and which is excellent in durability to friction, and even after washing, these effects can be sustained. In the case where the cosmetic composition is used as a hair dye composition, it can give a good feel to hair in any of a dry state and a wet state, and after hair dyeing, the color film peels little by friction, and can suppress secondary adhesion to clothes and pillows. In addition, after shampooing, discoloration is low and color sustainability is good, and further, even after shampooing, a good feel retains and a secondary adhesion suppressing effect can also be sustained.

## Claims

1. A cosmetic composition comprising the following components (A) to (C),

(A) a silicone film-forming agent,
(B) a high-molecular-weight organopolysiloxane,
(C) an organopolysiloxane having a polyalkylene oxide moiety and a cationic group, other than the component (A) and the component (B).

2. The cosmetic composition according to claim 1, wherein the component (A) is at least one selected from the group consisting of the following components (A1) and (A2):

(A1) a silicone resin represented by an average formula, $(R^1)_mSiO_{(4-m)/2}$

wherein $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3,
which contains at least one unit selected from the group consisting of a T unit represented by $R^1SiO_{3/2}$ and a Q unit represented by $SiO_{4/2}$,

(A2) a silicone polymer containing a polysiloxane moiety and a moiety formed of a non-silicone organic chain.

3. The cosmetic composition according to claim 2, wherein the component (A2) contains an acrylsilicone polymer (A2-1).

4. The cosmetic composition according to any one of claims 1 to 3, wherein the degree of polymerization of the component (B) is 650 or more.

5. The cosmetic composition according to any one of claims 1 to 4, wherein the component (B) is an organopolysiloxane represented by the following general formula (1):

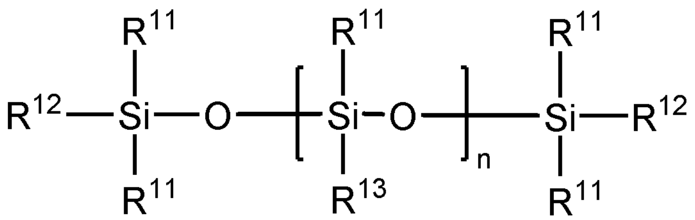
(1)

wherein $R^{11}$ each independently represents a hydrocarbon group having 1 or more and 6 or less carbon atoms, $R^{12}$ each independently represents a hydroxy group, an alkoxy group having 1 or more and 6 or less carbon atoms, or a hydrocarbon group having 1 or more and 6 or less carbon atoms, $R^{13}$ represents a hydrocarbon group having 1 or more and 6 or less carbon atoms, or a primary to tertiary amino group-containing group, n indicates a degree of polymerization, and is a number of 650 or more, and n's $R^{13}$'s can be the same as or different from each other.

6. The cosmetic composition according to any one of claims 1 to 5, wherein the alkylene oxide constituting the polyalkylene oxide moiety of the component (C) is at least one selected from ethylene oxide and propylene oxide.

7. The cosmetic composition according to any one of claims 1 to 6, wherein the ratio by mass of the content of the component (C) to the total content of the components (A), (B) and (C) in the cosmetic composition, [(C)/((A)+(B)+(C))], is 0.1% or more and 75% or less.

8. The cosmetic composition according to any one of claims 1 to 7, wherein the total content of the components (A) to (C) in the cosmetic composition is 0.1% by mass or more and 50% by mass or less.

9. The cosmetic composition according to any one of claims 1 to 8, further containing a functional powder as a component (D).

10. The cosmetic composition according to any one of claims 1 to 9, which is a hair cosmetic composition.

**11.** The cosmetic composition according to any one of claims 1 to 9, which is a hair dye composition.

**12.** A method for treating a keratin substance, comprising a step of applying the cosmetic composition of any one of claims 1 to 9 to a keratin substance and then drying it.

**13.** A method for treating hair, comprising a step of applying the hair cosmetic composition of claim 10 to hair and then drying it.

**14.** A method for dyeing hair, comprising a step of applying the hair dye composition of claim 11 to hair and then drying it.

**15.** A cosmetic kit provided with at least two compositions, wherein:
the cosmetic composition obtained by mixing all the compositions constituting the cosmetic kit contains the following components (A) to (C):

(A) a silicone film-forming agent,
(B) a high-molecular-weight organopolysiloxane,
(C) an organopolysiloxane having a polyalkylene oxide moiety and a cationic group, other than the component (A) and the component (B).

**16.** A method for forming a film on the surface of a keratin substance, comprising the following step (I) to step (III) in that order,

Step (I): a step of temporarily compatibilizing or dispersing a composition containing the following components (A) to (C),

(A) a silicone film-forming agent,
(B) a high-molecular-weight organopolysiloxane,
(C) an organopolysiloxane having a polyalkylene oxide moiety and a cationic group, other than the component (A) and the component (B),

Step (II): a step of applying the composition prepared in the step (I) to the surface of a keratin substance,
Step (III): a step of drying the composition applied to the surface of a keratin substance in the step (II).

**17.** A film to cover a surface of a keratin substance, wherein: the film is a multilayer-structured film containing the components (A) to (C), having a layer containing the component (B) in the outermost surface of the film, and having a layer containing the component (C) on the interface side to the keratin substance,

(A) a silicone film-forming agent,
(B) a high-molecular-weight organopolysiloxane,
(C) an organopolysiloxane having a polyalkylene oxide moiety and a cationic group, other than the component (A) and the component (B).

Fig. 1

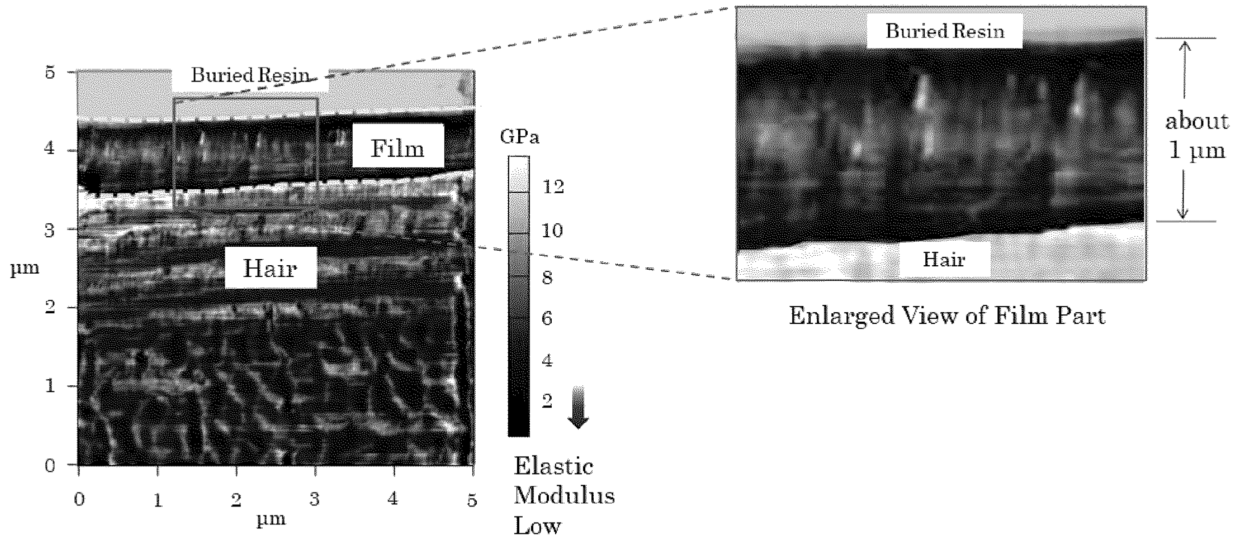
Buried Resin
Film
Hair
μm
0
1
2
3
4
5
GPa
12
10
8
6
4
2
Elastic
Modulus
Low
Buried Resin
Hair
about
1 μm
Enlarged View of Film Part

**INTERNATIONAL SEARCH REPORT**

International application No. PCT/JP2021/020130

A. CLASSIFICATION OF SUBJECT MATTER
A61K 8/89(2006.01)i; A61Q 5/00(2006.01)i; A61Q 5/06(2006.01)i
FI: A61K8/89; A61Q5/00; A61Q5/06

According to International Patent Classification (IPC) or to both national classification and IPC

B. FIELDS SEARCHED

Minimum documentation searched (classification system followed by classification symbols)
A61K8/89; A61Q5/00; A61Q5/06

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2008-106068 A (L'OREAL) 08 May 2008 (2008-05-08) paragraphs [0007]-[0008], [0016]-[0017], [0055], [0094], [0135], [0147]-[0150] | 1-17 |
| Y | JP 2020-7296 A (KAO CORP.) 16 January 2020 (2020-01-16) claims, paragraphs [0005]-[0036] | 1-17 |
| Y | JP 2018-111659 A (JO COSMETICS CO., LTD.) 19 July 2018 (2018-07-19) paragraphs [0019]-[0020] | 1-17 |
| Y | JP 2019-6691 A (KAO CORP.) 17 January 2019 (2019-01-17) paragraphs [0007], [0016] | 1-17 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 July 2021 (21.07.2021) | 10 August 2021 (10.08.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/020130

| Patent Documents referred in the Report | Publication Date | Patent Family | Publication Date |
|---|---|---|---|
| JP 2008-106068 A | 08 May 2008 | US 2008/0127429 A1 paragraphs [0008]-[0009], [0016]-[0017], [0066], [0137], [0224], [0245]-[0247]<br>EP 1935404 A1 | |
| JP 2020-7296 A | 16 Jan. 2020 | WO 2020/008812 A1 | |
| JP 2018-111659 A | 19 Jul. 2018 | WO 2018/131534 A1 | |
| JP 2019-6691 A | 17 Jan. 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015515981 T
- JP 2016503433 T
- JP 2000501074 T
- JP H10265354 A
- JP H111530 A
- JP 2000063225 A
- JP H225411 A
- JP H2132141 A
- JP H3162442 A
- JP 2003104825 A
- JP H692825 A